# EUROPEAN PATENT SPECIFICATION

(11) **EP 1 554 382 B1**
(45) Date of publication and mention of the grant of the patent: **22.04.2009**
(21) Application number: 03756576.9
(22) Date of filing: 17.10.2003
(51) Int. Cl.: C12N 15/12, C07K 14/72, A01K 67/027, G01N 33/68, A61K 38/17, A61K 48/00

(54) **GPR54 KNOCK-OUT MAMMALS AND SCREENING METHODS USING THEM**
GPR54 'KNOCK-OUT' SÄUGETIERE UND SCREENING-VERFAHREN, DIE SIE VERWENDEN
MAMMIFERES INACTIVES GPR54 ET PROCEDES DE CRIBLAGE UTILISANT CES MAMMIFERES

(30) Priority: 25.10.2002 GB 0224926; 20.06.2003 GB 0314481; 15.08.2003 US 495340 P; 03.09.2003 GB 0320623; 03.09.2003 US 499963 P
(43) Date of publication of application: 20.07.2005
(73) Proprietor: Takeda Pharmaceutical Company Limited, Osaka 541-0045 (JP)
(72) Inventor: APARICIO, Samuel c/o Paradigm Therapeutics Limited, Cambridge CB4 0GP (GB); COLLEDGE, William c/o Paradigm Therapeutics Ltd., Cambridge CB4 0GP (GB); DIXON, John c/o Paradigm Therapeutics Limited, Cambridge CB4 0GP (GB); HENDRICK, Alan c/o Paradigm Therapeutics Limited, Cambridge CB4 0GP (GB); MESSAGER, Sophie c/o Paradigm Therapeutics Limited, Cambridge CB4 0GP (GB); THRESHER, Rosemary c/o Paradigm Therapeutics Ltd., Cambridge CB4 0GP (GB); ZAHN, Dirk c/o Paradigm Therapeutics Limited, Cambridge CB4 0GP (GB)
(74) Representative: Jones, Nicholas Andrew
(86) International application number: PCT/GB2003/004479
(87) International publication number: WO 2004/038021

(56) References cited:
- WO-A-01/42486
- WO-A-02/059344
- US-A1- 2002 106 766
- KOTANI MASATO ET AL: "The metastasis suppressor gene KiSS-1 encodes kisspeptins, the natural ligands of the orphan G protein-coupled receptor GPR54" JOURNAL OF BIOLOGICAL CHEMISTRY, vol. 276, no. 37, 14 September 2001 (2001-09-14), pages 34631-34636, XP002262291 ISSN: 0021-9258 cited in the application
- OHTAKI T ET AL: "METASTASIS SUPPRESSOR GENE KISS-1 ENCODES PEPTIDE LIGAND OF A G-PROTEIN-COUPLED RECEPTOR" NATURE, vol. 411, 31 May 2001 (2001-05-31), pages 613-617, XP002942231 ISSN: 0028-0836 cited in the application

## Description

The present invention relates to novel functions of a known receptor. In particular, the invention relates to the use of a galanin-like receptor and/or ligands thereof in the manipulation of the pituitary hormonal axis and reproductive systems.

Harry potter, which encodes GPR54 is located on human chromosome 19p13.3.

GPR54 cDNA is 1197 bp long. Studies have shown that GPR54 is 45% identical to the rat galanin receptors GalR1, GalR2, and 44% to GalR3.

Galanin is a neuropeptide which is not a member of any known family of neuropeptides. It is expressed in both the central and the peripheral nervous systems and modulates a wide variety of physiological processes, including learning and memory, nociception, feeding, neurotransmitter and hormone release and sexual behaviour, and is considered to be involved in the pathogenesis of Alzheimer's disease (Lee et al 1999 FEBS lett 446: 103-107). However, it has been reported that neither galanin nor galanin-like peptide activate GPR54 (Lee et al 1999 FEBS lett 446: 103-107; Ohtaki et al 2001 Nature 411:613-617).

Reports suggest that FMRFamide-related peptides (FaRPs) are low potency agonists of GPR54 (micromolar range)(Clements et al 2001 Biochem Biophys Res Commun 284: 1189-83). FaRPS are neuropeptides abundantly distributed in invertebrates were they function as neurotransmitters and neuromodulators.

Further studies indicate that the natural ligand of GPR54 are kisspeptins (also called metastin), the products of the Kiss-1 gene. Three peptides with a common RF-amide C terminus derive from Kiss-1. There are a 54, 14 and 13 amino acid kisspeptins. They all bind GPR54 at low nanomolar affinities. Kisspeptin 54 is also called metastin. These GPR54 ligands bind GPR54 at low nanomolar affinities (Muir et al 2001 J Biol Chem 276:28969-75; Kotani et al 2001 J Biol Chem 276:34631-36).

Several reports have suggested various functional roles for ligand bound GPR54. For example, it has been reported that Kiss-1 is a metastasis suppressor gene for melanoma and breast carcinoma cells. However, studies suggest that the Kiss-1 gene does not affect tumorigenicity. It has been hypothesised that metastin attenuates cellular motility by excessively inducing the adhesive phenotypes of cells. In mice which have had melanomas injected in their footpads, administration of metastin by perfusion significantly decreased metastases (Ohtaki et al 2001 Nature 411:613-617). Similarly, metastin inhibits motility in B16-L16 melanoma cells transfected with the GPR54 receptor, but not in mock-transfected cells.

Further studies have shown that GPR54 (also called metastin receptor) activation stimulates PIP2 hydrolysis, calcium mobilisation, arachidonic acid release, ERK1/2 and p38 MAP kinase phosphorylation, and stress fibres formation but inhibits cell proliferation (Kotani et al 2001 J Biol Chem 276:34631-3). Interestingly, intravenous injection of kisspeptin-10 in female rat stimulates oxytocin secretion (Kotani et al 2001 J Biol Chem 276:34631-3). Oxytocin has been shown to be involved in sexual behaviour/testicular function; oxytocin antagonists inhibit male copulatory behaviour (Argiolas et al 1988 Eur J Pharmacol 149:389-92). However, oxytocin KO mice display normal sexual behaviour and are fertile (Nishimori et al 1996 Proc Natl Acad Sci USA 93:11699-704).

In the rat, GPR54 has been detected in the following tissues by northern blotting: brain (pons, midbrain, thalamus, hypothalamus, hippocampus, amygdala, cortex, frontal cortex and stratium), and peripheral organs (liver and intestine) (Lee et al 1999 FEBS lett 446: 103-107).

In situ analysis in the rat brain shows highest expression in the hypothalamic and amygdaloid areas. The receptor is highly expressed in zona incerta, ventral tegmental area, dentate gyrus, arcuate nucleus, dorsomedial nucleus, olfactory cortex, lateral habenular nucleus, lateral hypothalamus, locus coeruleus, cortical and medial nuclei of amygdala, superior colliculus, preoptic, anterior and posterior hypothalamus, periaqueducal gray, parafascicular thalamic nucleus, parabrachial nucleus and ventral mamillary body. GPR54 expression was found to ressemble that of galanin receptors (Lee et al 1999 FEBS lett 446: 103-107).

In human tissues, northern analysis shows expression in brain/nervous system (cortex, putamen, medulla, spinal cord, and lower expression in hippocampus, thalamus) and in pituitary, heart, skeletal muscle, kidney, liver, stomach, small intestine, thymus, lung, testis and placenta (Clements et al 2001 Biochem Biophys Res Commun 284: 1189-93; Kotani et al 2001 J Biol Chem 276:34631-36).

GPR54 is also detected by immunocytochemistry (ICC) in human brain in the following areas: cerebral cortex (pyramidal cells and their ascending processes in laminar layer III of sensory motor cortex), thalamus, pons-medulla (raphe, inferior olive, hypoglossal nuclei), cerebellum (purkinje cells perikarya and their ascending apical dendrites).

However, an understanding of the roles performed by GPR54 in mammals in general and particularly in the brain has not been achieved in the prior art.

Reports indicate that the vertebrate receptors for FaRPs (low potency agonists of GPR54 which function in the micromolar range) are most similar to receptors for neuropeptide Y (NPY) at the amino acid level (Clements et al 2001 Biochem Biophys Res Commun 284: 1189-93). Further studies have shown that FaRPs colocalise with gonadotropin-releasing hormone (GnRH, colocalise with spermatheca in locusta and with salivary glands in blood feeding bug

The Drosophila FMRFamide gene was identified by its homology to a gene first sequenced in the marine snail Aplysia. FMRFamide was first purified as a cardioregulatory tetrapeptide from the central nervous system of the clam. In molluscs, it modulates both cardiac output and the actions of neurons, as well as regulating evoked muscle tension. In cattle, two peptides immunoreactive to a related lobster hormone have been identified; the cattle proteins have anti-analgesic properties.

Although the role of FMRF-amide peptide has mainly be studied in invertebrates, there are 3 papers describing FmRF amide immunoreactivity in the brain of mammals: one in rabbit, rat and guinea pig (Duann et al 1995 Gaoxiong Yi Xue Ke Xue Za Zhi. 11:142-9), one in the tree shrew (Malz and Kuhn 2002 Dev Brain Res 135:39-44), and one in the big brown bat (Oelschlager et al 1998 Brain Behav Evol 52:139-47). Interestingly, all of them describe co-expression of FmRFamide-like and GnRH immunoreactivity. In the bat and tree shrew, this expression is described in the terminal nerve. The terminal nerve is an anterior cranial nerve that innervate the chemosensory epithelia of the nasal cavity. The function of this nerve is unknown, but it has been suggested to have a neuromodulatory role. Damage to this nerve impairs mating behaviour in the male hamster (Wirsig, and Leonard 1987 Brain Res 417:293-303).

According to Raffa (Raffa and Stone 1988 Peptides 19: 1171-75), FMRFamide, or FMRFamide-related peptides (FaRPs), are present in mammalian central nervous system and gastrointestinal tract and have been shown to be cardioexcitatory in mammals, to inhibit morphine-induced antinociception, and to block morphine-, defeat-, and deprivation-induced feeding. It also inhibits colonic propulsive motility, induces behavioural effects when administered intrathecally, and has been reported to have amnesic effects in rodents. In particular, FMRF amide produces centrally mediated antinociception in mice (Raffa and Stone 1998 Peptides 19: 1171-75, Gupta et al 1999 Peptides 20: 471-78).

Previous studies have shown that galanin knock out mice (no receptor knock-out has yet described) are viable, grow normally and can reproduce but not lactate. The response of their sensory neurones to injury is impaired, peripheral nerve regeneration is reduced, and they have a strong reduction in the development of neuropathic pain. A subset of DRG neurons is lost in the mutant mice which implies a role for galanin in cell survival (Wynick et al 1998 Ann NY Acad Sci, 863:22-47)

Studies have indicated that GPR54 also plays a role in metastasis involves a multistep process including detachment of cancer cells from primary cancer, invasion of surrounding tissue, spread through circulation, re-invasion and proliferation in distant organs. However, at the time of filing the present application no further roles of GPR54 have been identified.

Therefore, there remains a need in the art to identify the functions of ligand bound GPR54 both in the brain and other tissues, for therapeutic and other uses.

### SUMMARY OF THE INVENTION

The present inventors have created GPR54 (Harry Potter) knockout mice in order to investigate the *in vivo* role of GPR54. The inventors have shown that such knockout mice exhibit differences from non-mutants in their reproductive morphology and physiology that suggests a role for GPR54 in controlling the sex hormone axis and in reproductive areas such as controlling fertility and libido. In addition, the physiology and morphology of such mutants suggests a role for GPR54 in certain neurological disorders and other conditions for example muscle wasting and inflammation.

Harry Potter knockout mice are useful as models of disease and in identifying antagonists and agonists of GPR54 for therapeutic use.

Thus, in a first aspect, the present invention provides a GPR54 knockout mammal comprising one or more cells in which GPR54 polypeptide is functionally inactivated.

The present inventors have found that GPR54 knockout mice according to the above aspect of the invention show several defining characteristics including those selected from the group consisting of the following: reduced contact righting reflex and lower Barnes maze performance, alterations in reproductive hormone levels and/or patterns (including cycles), alterations in reproductive organ and associated organ morphology, alterations in sexual/reproductive behaviour.

As referred to above, alterations in reproductive organ morphology includes any one or more of those selected from the group consisting of the following: atrophy of genitals and associated organs, atrophy of stomach and sub maxillary salivary glands, atrophy of muscle mass and brown fat.

The generation of non-human knockout mammals will be familiar to those skilled in the art and uses standard laboratory techniques which involves homologous recombination as described herein.

Preferably, the transgenic non-human mammal is any one of the following non-human mammals selected from the group consisting of the following: mouse, rat, shrew, gerbil, guinea-pig, monkey, hamster, cat and dog. Those skilled in the art will appreciate that this list is not intended to be exhaustive. Most advantageously, the non-human mammal is a mouse.

As referred to herein, the term to 'functionally inactivated' (GPR54 by homologous recombination) means that one or more of the functions normally performed by GPR54 polypeptide when it is functioning in its native environment (that is within an in vivo environment) is significantly inhibited as compared with a control in which GPR54 has not been functionally inactivated. Advantageously, the term to 'functionally inactivate' means that more than one of the functions normally performed by GPR54 when it functioning in its native environment (that is within an in vivo environment) is significantly inhibited as compared with a control in which GPR54 has not been functionally inactivated. Most advantageously, as referred to herein, the term 'functionally inactivated' means that all of the functions normally performed by GPR54 when it functioning in its native environment is significantly inhibited as compared with a control in which GPR54 has not been functionally inactivated.

Likewise, 'functionally inactivated' GPR54 nucleic acid as herein defined encodes functionally inactive GPR54 as herein defined.

As referred to above, the term 'significantly inhibited' (GPR54 function) means that the inhibition (of at least one GPR54 function in a mammalian cell by homologous recombination, as described above) is inhibited by at least 20% as compared with suitable control. An example of a suitable control is the same or a similar cell in the same or similar in vivo environment wherein GPR54 has not been functionally inactivated by homologous recombination as herein described. Advantageously, the term 'significantly inhibited' (GPR54 function in a mammalian cell by homologous recombination, as described above) means that at least one GPR54 function is inhibited by at least 30%, 40%, 50%, 60%, 70%, 80%, 90%, 95% as compared with a suitable control. Most advantageously, the term 'significantly inhibited' (GPR54 function in a mammalian cell by homologous recombination, as described above) means that at least one GPR54 function is inhibited by 100% as compared with a suitable control.

In a further aspect, the present invention provides a method for generating one or more mammalian cells comprising one or more functionally inactive GPR54 gene comprising the steps of:
selecting one or more cells comprising one or more functionally active endogenous GPR54 gene/s.

Transfecting the one or more cells according to step (a) with functionally inactive GPR54 nucleic acid which is capable of recombining by homologous recombination with the one or more endogenous GPR54 genes.

Selecting those one or more cells in which the one or more endogenous GPR54 genes have undergone homologous recombination with the functionally inactive GPR54 nucleic acid.

Advantageously, the mammalian cells according to the invention are generated according to the methods of the invention detailed above. In a preferred embodiment of this aspect of the invention, the mammalian cells are comprised within a non-human mammal. That is the cells according to the above aspect of the invention will preferably constitute a non-human knock-out' mammal. Advantageously, the non-human knock-out mammal according to the present invention is a mouse.

Methods for transfecting those one or more cells with functionally inactive GPR54 involve the use of standard molecular biology techniques and are described herein. Likewise, methods for selecting those one or more cells in which the one or more endogenous GPR54 genes have undergone homologous recombination with the functionally inactive GPR54 nucleic acid are performed using standard laboratory techniques, which are described herein.

In a further aspect, the present invention provides a nucleic acid construct suitable for functionally inactivating one or more endogenous GPR54 genes in a host cell comprising:
(a) A non-homologous replacement region
(b) A first homology region located upstream of the non-homologous replacement region
(c) A mutated GPR54 gene and which when expressed does not encode functionally active GPR54.
(d) A second homology region located downstream of the non-homologous replacement portion, the second homology region located downstream of the non-homologous replacement region, the second homology region having a nucleotide sequence exhibiting at least 90% identity to a second GPR54 gene.

The present inventors have identified several functions associated with GPR54, thus they consider that GPR54 polypeptides, or one or more binding protein/s thereof or the nucleic acid encoding them may be of therapeutic significance.

Thus, in a further aspect still, the present invention provides a composition comprising GPR54 polypeptides, or one or more binding protein/s thereof or the nucleic acid encoding them, and a pharmaceutically acceptable carrier, diluent or exipient.

According to the above aspect of the invention, advantageously the composition comprises one or more GPR54 polypeptide binding protein/s. Preferably the one or more GPR54 binding proteins are antagonists or agonists of GPR54.

GPR54 binding proteins may be naturally occurring or synthetic. Naturally occurring binding proteins may be polypeptides such as antibodies as herein defined, or nucleic acids. Synthetic GPR54 binding proteins are advantageously small molecules and may be selected by screening methods which will be familiar to those skilled in the art and are described herein.

In an alternative embodiment of the invention, preferably the composition comprises nucleic acid encoding a GPR54 binding protein or nucleic acid encoding GPR54 polypeptide.

The present inventors have surprisingly found that GPR54 knockout mice have altered neuronal function as well as altered reproductive systems as compared with normal control mice. In addition GPR54 knockout mice possess other morphological and anatomical abnormalities. The defect in GPR54 knockout mice is at the pituitary hypothalamic axis in production of gonadotrophs. GPR54 receptors are thus believed to be involved in controlling this axis, through control of the pituitary-hypothalamic secrection of gonadotrophs. The present inventors have shown that, in GPR54 knockout mice:
(a) gonadotrophins are low;
(b) at least ovaries respond to administration of exogenous gonadotrophins, indicating that they retain the capacity to respond; and
(c) exogenous administration of GnRH provokes at least a partial response in secretion of FSH and LH, indicating that GPR54 may be involved in the upstream control of GnRH secretion in the hypothalamus-pituitary.

Thus, the inventors consider that agonists and/or antagonists of GPR54 or the compositions comprising them may be of particular therapeutic use in the treatment of reproductive hormone related, neurological, and certain other conditions. Such neurological conditions include but are not limited to any one or more of the following: Alzheimers, sensory neuropathies and epilepsy.

Such reproductive hormone related conditions consist of any one or more of the group consisting of the following: the modulation of fertility, contracteption, libido and the onset of puberty, lactation, osteoporosis/osteopetrosis and menopause. In addition, GPR54 agonists or antagonist may be used in the useful for hormone replacement therapy

Other conditions include muscle wasting diseases, pain, hormone dependent cancer and obesity.

Thus, in a further aspect the present invention provides a method of identifying one or more molecules which agonise the functional activity of GPR54 polypeptide comprising the steps of :
(a) selecting one or more non-human mammals comprising functionally inactive GPR54 polypeptide;
(b) treating those one or more non-human mammals with one or more potential GPR54 mimics which potentially agonise at least on aspect of GPR54 activity;
(c) testing those one or more non-human mammals treated according to step (b) to determine if those treated non-human mammals have one or more restored characteristics compared with those non-human mammals selected according to step (a) and
(d) selecting those one or more GPR54-ligand mimics which restore one or more characteristics of wild type non-human mammals to those non-human mammals selected according to step (a).

The invention further provides method of identifying one or more molecules which antagonise the functional activity of GPR54 polypeptide comprising the steps of:
(a) selecting one or more non-human mammals comprising functionally active GPR54 polypeptide;
(b) treating those one or more non-human mammals with one or more potential GPR54 antagonists which potentially antagonise at least on aspect of GPR54 activity;
(c) testing those one or more non-human mammals treated according to step (b) to determine if those treated non-human mammals have one or more modulated characteristics compared with those non-human mammals selected according to step (a).

Moreover, the invention provides the use of a transgenic non-human GPR54 knockout mammal in an assay for a biological effect of one or more compounds.

According to the above aspects of the invention, the term 'antagonist' refers to a molecule which significantly inhibits as herein defined one or more functions of GPR54 as compared with a suitable control.

The invention makes use of GPR54 itself, agonists and antagonists thereof, as well as modulators of GPR54 activity. Those skilled in the art will recognise that various agents will act to increase or decease the effect of GPR54, and that the routes through which this is achieved will vary; thus, agonists may mimic activated GPR54, or bind to GPR54 and increase its activity; agonistic modulators of GPR54 activity may do the same, or increase the production of endogenous agonists of GPR54 or endogenous GPR54 itself. Antagonists and antagonistic modulators may act likewise, but to decrease the biological effect of GPR54.

As referred to herein the term, 'mammal' may be any non-human mammal. Preferably, the non-human mammal is any selected from the group consisting of the following: mouse, rat, guinea-pig, rabbit, hamster, gerbil, cat, dog and monkey. Those skilled in the art will appreciate that this list is not intended to be exhaustive.

According to this aspect of the invention, the term 'treating' (the mammal with a potential antagonist of GPR54) means to bring one or more cells of the mammal into contact (with one or more potential antagonists of GPR54).

According to the above aspect of the present invention, indicators of aberrant neurological characteristics include any of those selected from the group consisting of the following: reduced contact righting reflex and lower Barnes maze performance.

According to the above aspect of the invention, the term 'aberrant reproductive systems and/or morphology' includes within its scope any alterations in reproductive hormone levels and/or patterns (including cycles) as compared with suitable controls, any alterations in reproductive organ and associated organ morphology as compared with suitable controls and alterations in sexual/reproductive behaviour as compared with suitable controls.

Advantageously, according to the above aspect of the invention, alterations in reproductive organ morphology as compared with suitable controls includes any one or more of those selected from the group consisting of the following: atrophy of genitals and associated organs, atrophy of stomach and sub maxillary salivary glands, atrophy of muscle mass and brown fat.

Tests for aberrant neurological function, aberrant reproductive systems and aberrant reproductive morphology utilise standard laboratory techniques and will be familiar to those skilled in the art.

Advantageously, step (c) of the above aspect of the invention involves testing those one or more non-human mammals to determine if those non-human mammals exhibit one or more characteristics exhibited by GPR54 knockout mice and which are aberrant reproductive systems and/or morphology.

According to the above aspect of the invention, the one or more non-human mammals selected according to step (a) are advantageously GPR54 knockout mice. Preferably, they are generated according to the methods herein described.

As referred to herein, the term 'functionally inactive' (GPR54) means that one or more of the functions normally performed by GPR54 polypeptide when it is functioning in its native environment (that is within an in vivo environment) is significantly inhibited as compared with a control in which GPR54 is not functionally inactive. Advantageously, the term to 'functionally inactive' means that more than one of the functions normally performed by GPR54 when it functioning in its native environment (that is within an in vivo environment) is significantly inhibited as compared with a control in which GPR54 has not been functionally inactivated. Most advantageously, as referred to herein, the term 'functionally inactive' means that all of the functions normally performed by GPR54 when it is functioning in its native environment is significantly inhibited as compared with a control in which GPR54 has not been functionally inactivated.

Likewise, 'functionally inactive' GPR54 nucleic acid as herein defined encodes functionally inactive GPR54 as herein defined.

As referred to above, the term 'significantly inhibited' (GPR54 function) means that the inhibition (of at least one GPR54 function in a mammalian cell by homologous recombination, as described above) is inhibited by at least 20% as compared with suitable control. An example of a suitable control is the same or a similar cell in the same or similar in vivo environment wherein GPR54 has not been functionally inactivated by homologous recombination as herein described. Advantageously, the term 'significantly inhibited' (GPR54 function in a mammalian cell by homologous recombination, as described above) means that at least one GPR54 function is inhibited by at least 30%, 40%, 50%, 60%, 70%, 80%, 90%, 95% as compared with a suitable control. Most advantageously, the term 'significantly inhibited' (GPR54 function in a mammalian cell by homologous recombination, as described above) means that at least one GPR54 function is inhibited by 100% as compared with a suitable control.

According to this aspect of the invention, the term 'treating' (the mammal with a potential antagonist of GPR54) means to bring one or more cells of the non-human mammal into contact (with one or more potential antagonists of GPR54).

According to the above aspect of the invention, the term 'one or more modulated characteristics of non-human wild type mammals' refers to the modulation of one or more characteristics shown by non-human wild type mammals as compared with non-human mammals comprising functionally inactive GPR54 polypeptide as herein defined. Preferably the modulation is the restoration of a lost function.

GPR54 mimics, as referred to herein, replicate at least one activity or function of wild-type GPR54. The mimics may mimic activated GPR54, or may mimic inactive GPR54 such that they further repress functions which are themselves repressed in the absence of natural inactive GPR54.

The inventors consider that GPR54 nucleic acid or nucleic acid encoding one or more agonists or antagonists of GPR54 may be inserted into a non-human mammal in order to generate one or more therapeutic effects.

Thus in a further aspect, the present invention provides a non-human transgenic animal comprising within at least a proportion of its cells, exogenous nucleic acid encoding one or more selected from the group consisting of the following: GPR54 polypeptide, one or more agonists of GPR54 polypeptide and one or more antagonists of GPR54 polypeptide.

Advantageously, the non-human transgenic animal is selected from the group consisting of the following: a mouse, pig, goat, deer, monkey and cow. Those skilled in the art will appreciate that this list is not intended to be exhaustive.

According to the above aspect of the invention, the term 'exogenous nucleic acid' means nucleic acid which does not originate from that specific animal. It may however originate from that same animal type or breed. For example, a transgenic mouse may comprise GPR agonist nucleic acid of bacterial origin.

Preferably, a non-human transgenic animal comprises within a proportion of its cells, exogenous DNA encoding one or more agonists or one or more antagonists of GPR54

As herein described, the non-human transgenic animal may be any one or more selected from the group consisting of: mouse, rat, monkey, dog, cat and pig. Those skilled in the art will appreciate that this list is not intended to be exhaustive.

Studies using GPR54 knockout mice prepared according to the present invention have shown that the GPR54 polypeptide is associated with neuronal function as well as reproductive function (including reproductive cycles) as well as being associated with other conditions. Such neurological conditions include but are not limited to any one or more of the following: Alzheimers, sensory neuropathies and epilepsy. Such reproductive hormone related conditions consist of any one or more of the group consisting of the following: the modulation of fertility, libido and the onset of puberty, lactation, osteoporosis/osteopetrosis and menopause. In addition, GPR54 agonists or antagonists may be used in the useful for hormone replacement therapy (HRT). Other conditions include muscle wasting diseases, pain, hormone dependent cancer and obesity.

Thus, in a further aspect, the present invention provides a method for the diagnosis of a disease or condition selected from the group consisting of the following: Alzheimers, sensory neuropathies and epilepsy, the modulation of fertility, libido and the onset of puberty, lactation, osteoporosis/osteopetrosis, menopause, muscle wasting diseases, pain, hormone dependent cancer and obesity comprising the steps of:
selecting a sample of cells from a patient to be diagnosed,
comparing the expression levels and/or functional activity of GPR54 polypeptide in those sample of cells with one or more control sample/s from healthy individuals.

In a further embodiment, polymorphisms in Harry Potter or its natural ligands may be used to diagnose disease. Thus, the present invention provides a method for the diagnosis of a disease or condition selected from the group consisting of the following:
Alzheimers, sensory neuropathies and epilepsy, the modulation of fertility, libido and the onset of puberty, lactation, osteoporosis/osteopetrosis, menopause, muscle wasting diseases, pain, hormone dependent cancer and obesity comprising the steps of:
   selecting a sample of cells from a patient to be diagnosed, and
   analysing the endogenous nucleic acids in said cells to detect one or more polymorphisms in the endogenous GPR54 gene one or more natural ligands of GPR54.

According to the above aspect of the invention, those samples in which the expression levels and/or functional activity of GPR54 is increased, decreased or otherwise altered, or in which GPR54 nucleic acids or nucleic acids encoding GPR54 ligands comprise one or more polymorphisms, as compared with one or more control samples from healthy individuals indicates that the individuals from which the samples are taken have any one or more of the diseases listed above.

According to the above aspect of the invention the sample of cells from a patient may be selected using standard laboratory techniques, which will be familiar to those skilled in the art.

As referred to above the term 'functional activity' of GPR54 polypeptide refers to the function GPR54 normally performs in its native environment, that is within its cell.

Thus, in a further aspect still, the present invention provides a method for the treatment of a condition in a patient selected from the group consisting of: muscle wasting diseases, pain, inflammation, hormone dependent cancer, lactation, osteoporosis/osteopetrosis, hormone imbalances related to the menopause and obesity comprising the step of treating the patient with a therapeutically effective amount of one or more antagonists or agonists of GPR54.

In yet a further aspect, the present invention provides the use of one or more agonist or antagonists of GPR54 in the preparation of a medicament for the prophylaxis or treatment of a condition in a patient selected from the group consisting of: muscle wasting diseases, pain, inflammation, hormone dependent cancer, lactation, osteoporosis/osteopetrosis, hormone imbalances related to the menopause and obesity.

In a further aspect still, the present invention provides a method for manipulating the sex hormone axis in a patient comprising the step of treating the patient with a therapeutically effective amount of one or more antagonists or agonists of GPR54.

In yet a further aspect, the present invention provides the use of an agonist or antagonist of GPR54 in the preparation of a medicament for manipulating the sex hormone axis in an animal.

According to the above two aspects of the invention, preferably manipulating the sex hormone axis results in the manipulation of any one or more effects or conditions selected from the group consisting of the following: fertility, libido, contraception and precocious puberty.

### BRIEF DESCRIPTION OF THE FIGURES

Figure 1 shows the structure of the genomic locus of mouse GPR54 before GPR54 knock-out
Figure 2 shows the structure of the genomic locus of mouse GPR54 after GPR54 knock-out.
Figure 3 shows the structure of a targeting vector used for homologous recombination of GPR54, including the relevant restriction sites.
Figure 4a shows a knockout mouse brain slice and Figure 4b heterozygote and wild type testes.
Figure 5 a show the testes of wild type and mutant mice. Figure 5b shows the preputial glands of wildtype mice and Figure 5c mutants.
Figure 6 shows the ovaries for wild type (top) and knock-out mice (bottom).
Figure 7 shows a graph of the results of the Barnes maze test comparing behaviour of the wild type and knock-out mice.
Figure 8 shows pictures of the vaginal smears of knock-out mice.
Figure 9 shows a graph of the mean testis weight of wildtype and nock-out mice
Figure 10 shows a graph of the mean muscle weight of wildtype and nock-out mice
Figure 11 shows a graph of the mean adrenal gland (Figure 11a) and salivary (Figure 11b) weight of wildtype and nock-out mice.
Figure 12 shows a graph of testosterone levels from male and female GPR54 Knock-out mice compared to wildtype mice.
Figure 13 shows a graph of oestradiol levels in female knock-out mice.
Figure 14 shows a graph of FSH levels in females (Figure 14a) and males (Figure14b) knock-out mice.
Figure 15 shows a graph of LH levels in serum (Figure15a) and in pituitary (Figure 15b)
Figure 16 shows an electronic Northern.

### DETAILED DESCRIPTION OF THE INVENTION

### GENERAL TECHNIQUES

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press.

### GPR54

For the avoidance of doubt, the term "GPR54" should be taken in this document to refer to any of the following sequences, identified by the relevant GenBank accession numbers:

NT_011255 Homo sapiens chromosome 19 genomic contig; NM_053244 Mus musculus G protein-coupled receptor 54 (Gpr54), mRNA; BC016531 Mus musculus G protein-coupled receptor 54, mRNA (cDNA clone MGC:27839 IMAGE:3488082), complete cds; M_032551 Homo sapiens G protein-coupled receptor 54 (GPR54), mRNA; NM_023992 Rattus norvegicus G protein-coupled receptor 54 (Gpr54), mRNA; NW_047773 Rattus norvegicus chromosome 7 WGS supercontig; AK039628 Mus musculus adult male spinal cord cDNA, RIKEN full-length enriched library, clone:A330075J02 product:G-PROTEIN-COUPLED RECEPTOR GPR54 (G PROTEIN-COUPLED RECEPTOR 54), full insert sequence; NT_039496 Mus musculus chromosome 10 genomic contig, strain C57BL/6J; AY029541 Homo sapiens putative G protein-coupled receptor mRNA, complete cds; AF343726 Mus musculus G-protein-coupled receptor GPR54 (Gpr54) mRNA, complete cds; AF343725 Homo sapiens G-protein-coupled receptor GPR54 (GPR54) mRNA, complete cds; BE506644 db65f08.yl Wellcome CRC pSK egg Xenopus laevis cDNA clone IMAGE:3377895 5' similar to TR:Q9Z0T7 Q9Z0T7 ORPHAN G PROTEIN-COUPLED RECEPTOR GPR54. ;, MRNA sequence; BB261471 BB261471 RIKEN full-length enriched, 7 days neonate cerebellum Mus musculus cDNA clone A730098N23 3' similar to AF115516 Rattus norvegicus orphan G protein-coupled receptor GPR54 (GPR54) mRNA, MRNA sequence; AF115516 Rattus norvegicus orphan G protein-coupled receptor GPR54 (GPR54) mRNA, complete cds.

### GPR54 POLYPEPTIDES

As herein described the term "Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids.

"Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications.

Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-inking, cyclization, disulfide bond formation, demethylation, formation of covalent cross-inks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins - Structure and Molecular Properties, 2nd Ed., T. E. Creighton, W. H. Freeman and Company, New York, 1993 and Wold, F., Posttranslational Protein Modifications: Perspectives and Prospects, pgs. 1-12 in Posttranslational Covalent Modification of Proteins, B. C. Johnson, Ed., Academic Press, New York, 1983; Seifter et al., "Analysis for protein modifications and nonprotein cofactors", Meth Enzymol (1990) 182:626-646 and Rattan et aL, "Protein Synthesis: Posttranslational Modifications and Aging", Ann NY Acad Sci (1992) 663:48-62.

The terms "variant", "homologue", "derivative" or "fragment" in relation to the present invention include any substitution of, variation of, modification of, replacement of, deletion of or addition of one (or more) amino acid from or to a sequence. Unless the context admits otherwise, references to "GPR54" and include references to such variants, homologues, derivatives and fragments of GPR54.

Where reference is made to the "receptor activity" or "biological activity" of a receptor such as GPR54, these terms are intended to refer to the metabolic or physiological function of the GPR54 receptor, including similar activities or improved activities or these activities with decreased undesirable side effects. Also included are antigenic and immunogenic activities of the GPR54 receptor. Examples of GPCR activity, and methods of assaying and quantifying these activities, are known in the art, and are described in detail elsewhere in this document.

As used herein a "deletion" is defined as a change in either nucleotide or amino acid sequence in which one or more nucleotides or amino acid residues, respectively, are absent. As used herein an "insertion" or "addition" is that change in a nucleotide or amino acid sequence which has resulted in the addition of one or more nucleotides or amino acid residues, respectively, as compared to the naturally occurring substance. As used herein "substitution" results from the replacement of one or more nucleotides or amino acids by different nucleotides or amino acids, respectively.

GPR54 polypeptides according to the present invention may also have deletions, insertions or substitutions of amino acid residues which produce a silent change and result in a functionally equivalent amino acid sequence. Deliberate amino acid substitutions may be made on the basis of similarity in polarity, charge, solubility, hydrophobicity, hydrophilicity; and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

### GPR54 NUCLEOTIDES AND POLYNUCLEOTIDES

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

It will be understood by the skilled person that numerous nucleotide sequences can encode the same polypeptide as a result of the degeneracy of the genetic code.

As used herein, the term "nucleotide sequence" refers to nucleotide sequences, oligonucleotide sequences, polynucleotide sequences and variants, homologues, fragments and derivatives thereof (such as portions thereof). The nucleotide sequence may be DNA or RNA of genomic or synthetic or recombinant origin which may be double-stranded or single-stranded whether representing the sense or antisense strand or combinations thereof. The term nucleotide sequence may be prepared by use of recombinant DNA techniques (for example, recombinant DNA).

Preferably, the term "nucleotide sequence" means DNA.

The terms "variant", "homologue", "derivative" or "fragment" in relation to the present invention include any substitution of, variation of, modification of, replacement of, deletion of or addition of one (or more) nucleic acids from or to the sequence of a GPR54 nucleotide sequence. Unless the context admits otherwise, references to "GPR54" and "GPR54" include references to such variants, homologues, derivatives and fragments of GPR54.

### EXPRESSION ASSAYS FOR GPR54

In order to design useful therapeutics for treating GPR54 associated diseases, it is useful to determine the expression profile of GPR54 (whether wild-type or a particular mutant). Thus, methods known in the art may be used to determine the organs, tissues and cell types (as well as the developmental stages) in which GPR54 is expressed. For example, traditional or "electronic" Northerns may be conducted. Reverse-transcriptase PCR (RT-PCR) may also be employed to assay expression of the GPR54 gene or mutant. More sensitive methods for determining the expression profile of GPR54 include RNAse protection assays, as known in the art.

Northern analysis is a laboratory technique used to detect the presence of a transcript of a gene and involves the hybridisation of a labelled nucleotide sequence to a membrane on which RNAs from a particular cell type or tissue have been bound. (Sambrook, supra, ch. 7 and Ausubel, F. M. et al. supra, ch. 4 and 16.) Analogous computer techniques ("electronic Northerns") applying BLAST may be used to search for identical or related molecules in nucleotide databases such as GenBank or the LIFESEQ database (Incyte Pharmaceuticals). This type of analysis has advantages in that they may be faster than multiple membrane-based hybridizations. In addition, the sensitivity of the computer search can be modified to determine whether any particular match is categorised as exact or homologous.

The polynucleotides and polypeptides of the present invention, including the probes described above, may be employed as research reagents and materials for discovery of treatments and diagnostics to animal and human disease, as explained in further detail elsewhere in this document.

### EXPRESSION OF GPR54 POLYPEPTIDES

The Expression of GPR54 polypeptides is required for the generation of GPR54 antibodies which may function as agonists or antagonists of GPR54 as described herein.

In order to express a biologically active GPR54 polypeptide, the nucleotide sequences encoding GPR54 or homologues, variants, or derivatives thereof are inserted into appropriate expression vector, i.e., a vector which contains the necessary elements for the transcription and translation of the inserted coding sequence.

Methods which are well known to those skilled in the art are used to construct expression vectors containing sequences encoding GPR54 and appropriate transcriptional and translational control elements. These methods include *in vitro* recombinant DNA techniques, synthetic techniques, and in vivo genetic recombination. Such techniques are described in Sambrook, J. et al. (1989; Molecular Cloning, A Laboratory Manual, ch. 4, 8, and 16-17, Cold Spring Harbor Press, Plainview, N.Y.) and Ausubel, F. M. et al. (1995 and periodic supplements; Current Protocols in Molecular Biology, ch. 9, 13, and 16, John Wiley & Sons, New York, N.Y.).

A variety of expression vector/host systems may be utilised to contain and express sequences encoding GPR54. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus (CaMV) or tobacco mosaic virus (TMV)) or with bacterial expression vectors (e.g., Ti or pBR322 plasmids); or animal cell systems. The invention is not limited by the host cell employed.

The "control elements" or "regulatory sequences" are those non-translated regions of the vector (i.e., enhancers, promoters, and 5' and 3' untranslated regions) which interact with host cellular proteins to carry out transcription and translation. Such elements may vary in their strength and specificity. Depending on the vector system and host utilised, any number of suitable transcription and translation elements, including constitutive and inducible promoters, may be used. For example, when cloning in bacterial systems, inducible promoters such as the hybrid lacZ promoter of the BLUESCRIPT phagemid (Stratagene, La Jolla, Calif.) or PSPorT1 plasmid (GIBCO/BRL), and the like, may be used. The baculovirus polyhedrin promoter may be used in insect cells. Promoters or enhancers derived from the genomes of plant cells (e.g., heat shock, RUBISCO, and storage protein genes) or from plant viruses (e.g., viral promoters or leader sequences) may be cloned into the vector. In mammalian cell systems, promoters from mammalian genes or from mammalian viruses are preferable. If it is necessary to generate a cell line that contains multiple copies of the sequence encoding GPR54 polypeptide, vectors based on SV40 or EBV may be used with an appropriate selectable marker.

In bacterial systems, a number of expression vectors may be selected depending upon the use intended for GPR54 polypeptide. For example, when large quantities of GPR54 polypeptide are needed for the induction of antibodies, vectors which direct high level expression of fusion proteins that are readily purified may be used. Such vectors include, but are not limited to, multifunctional E. coli cloning and expression vectors such as BLUESCRIPT (Stratagene), in which the sequence encoding GPR54 polypeptide may be ligated into the vector in frame with sequences for the amino-terminal Met and the subsequent 7 residues of β-galactosidase so that a hybrid protein is produced, pIN vectors (Van Heeke, G. and S. M. Schuster (1989) J. Biol. Chem. 264:5503-5509), and the like. pGEX vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast *Saccharomyces cerevisiae*, a number of vectors containing constitutive or inducible promoters, such as alpha factor, alcohol oxidase, and PGH, may be used. For reviews, see Ausubel (supra) and Grant et al. (1987; Methods Enzymol. 153:516-544).

In cases where plant expression vectors are used, the expression of sequences encoding GPR54 polypeptide may be driven by any of a number of promoters. For example, viral promoters such as the 35S and 19S promoters of CaMV may be used alone or in combination with the omega leader sequence from TMV. (Takamatsu, N. (1987) EMBO J. 6:307-311.) Alternatively, plant promoters such as the small subunit of RUBISCO or heat shock promoters may be used. (Coruzzi, G. et al. (1984) EMBO J. 3:1671-1680; Broglie, R. et al. (1984) Science 224:838-843; and Winter, J. et al. (1991) Results Probl. Cell Differ. 17:85-105.) These constructs can be introduced into plant cells by direct DNA transformation or pathogen-mediated transfection. Such techniques are described in a number of generally available reviews. (See, for example, Hobbs, S. or Murry, L. E. in McGraw Hill Yearbook of Science and Technology (1992) McGraw Hill, New York, N.Y.; pp. 191-196.).

An insect system may also be used to express GPR54 polypeptide. For example, in one such system, *Autographa californica* nuclear polyhedrosis virus (AcNPV) is used as a vector to express foreign genes in *Spodoptera frugiperda* cells or in *Trichoplusia* larvae. The sequences encoding GPR54 may be cloned into a non-essential region of the virus, such as the polyhedrin gene, and placed under control of the polyhedrin promoter. Successful insertion of GPR54 polypeptide will render the polyhedrin gene inactive and produce recombinant virus lacking coat protein. The recombinant viruses may then be used to infect, for example, *S*. *frugiperda* cells or *Trichoplusia* larvae in which GPR54 polypeptide may be expressed. (Engelhard, E. K. et al. (1994) Proc. Nat. Acad. Sci. 91:3224-3227.)

In mammalian host cells, a number of viral-based expression systems may be utilised. In cases where an adenovirus is used as an expression vector, sequences encoding GPR54 polypeptide may be ligated into an adenovirus transcription/translation complex consisting of the late promoter and tripartite leader sequence. Insertion in a non-essential E1 or E3 region of the viral genome may be used to obtain a viable virus which is capable of expressing GPR54 polypeptide in infected host cells. (Logan, J. and T. Shenk (1984) Proc. Natl. Acad. Sci. 81:3655-3659.) In addition, transcription enhancers, such as the Rous sarcoma virus (RSV) enhancer, may be used to increase expression in mammalian host cells.

Human artificial chromosomes (HACs) may also be employed to deliver larger fragments of DNA than can be contained and expressed in a plasmid. HACs of about 6 kb to 10 Mb are constructed and delivered via conventional delivery methods (liposomes, polycationic amino polymers, or vesicles) for therapeutic purposes.

Specific initiation signals may also be used to achieve more efficient translation of sequences encoding GPR54 polypeptide. Such signals include the ATG initiation codon and adjacent sequences. In cases where sequences encoding GPR54 polypeptide and its initiation codon and upstream sequences are inserted into the appropriate expression vector, no additional transcriptional or translational control signals may be needed. However, in cases where only coding sequence, or a fragment thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers appropriate for the particular cell system used, such as those described in the literature. (Scharf, D. et al. (1994) Results Probl. Cell Differ. 20:125-162.)

In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding, and/or function. Different host cells which have specific cellular machinery and characteristic mechanisms for post-translational activities (e.g., CHO, HeLa, MDCK, HEK293, and WI38), are available from the American Type Culture Collection (ATCC, Bethesda, Md.) and may be chosen to ensure the correct modification and processing of the foreign protein.

For long term, high yield production of recombinant proteins, stable expression is preferred. For example, cell lines capable of stably expressing GPR54 GPCR can be transformed using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase genes (Wigler, M. et al. (1977) Cell 11:223-32) and adenine phosphoribosyltransferase genes (Lowy, I. et al. (1980) Cell 22:817-23), which can be employed in tk⁻ or apr⁻ cells, respectively. Also, antimetabolite, antibiotic, or herbicide resistance can be used as the basis for selection. For example, dhfr confers resistance to methotrexate (Wigler, M. et al. (1980) Proc. Natl. Acad. Sci. 77:3567-70); npt confers resistance to the aminoglycosides neomycin and G-418 (Colbere-Garapin, F. et al (1981) J. Mol. Biol. 150:1-14); and als or pat confer resistance to chlorsulfuron and phosphinotricin acetyltransferase, respectively (Murry, supra). Additional selectable genes have been described, for example, trpB, which allows cells to utilize indole in place of tryptophan, or hisD, which allows cells to utilize histinol in place of histidine. (Hartman, S. C. and R. C. Mulligan (1988) Proc. Natl. Acad. Sci. 85:8047-51.) Recently, the use of visible markers has gained popularity with such markers as anthocyanins, β-glucuronidase and its substrate GUS, and luciferase and its substrate luciferin. These markers can be used not only to identify transformants, but also to quantify the amount of transient or stable protein expression attributable to a specific vector system. (Rhodes, C. A. et al. (1995) Methods Mol. Biol. 55:121-131.)

Although the presence/absence of marker gene expression suggests that the gene of interest is also present, the presence and expression of the gene may need to be confirmed. For example, if the sequence encoding GPR54 polypeptide is inserted within a marker gene sequence, transformed cells containing sequences encoding GPR54 polypeptide can be identified by the absence of marker gene function. Alternatively, a marker gene can be placed in tandem with a sequence encoding GPR54 polypeptide under the control of a single promoter. Expression of the marker gene in response to induction or selection usually indicates expression of the tandem gene as well.

Alternatively, host cells which contain the nucleic acid sequence encoding GPR54 polypeptide and express GPR54 may be identified by a variety of procedures known to those of skill in the art. These procedures include, but are not limited to, DNA-DNA or DNA-RNA hybridizations and protein bioassay or immunoassay techniques which include membrane, solution, or chip based technologies for the detection and/or quantification of nucleic acid or protein sequences.

The presence of polynucleotide sequences encoding GPR54 polypeptide can be detected by DNA-DNA or DNA-RNA hybridisation or amplification using probes or fragments or fragments of polynucleotides encoding GPR54 GPCR. Nucleic acid amplification based assays involve the use of oligonucleotides or oligomers based on the sequences encoding GPR54 polypeptide to detect transformants containing DNA or RNA encoding GPR54.

A variety of protocols for detecting and measuring the expression of GPR54, using either polyclonal or monoclonal antibodies specific for the protein, are known in the art. Examples of such techniques include enzyme-linked immunosorbent assays (ELISAs), radioimmunoassays (RIAs), and fluorescence activated cell sorting (FACS). A two-site, monoclonal-based immunoassay utilizing monoclonal antibodies reactive to two non-interfering epitopes on GPR54 is preferred, but a competitive binding assay may be employed. These and other assays are well described in the art, for example, in Hampton, R. et al. (1990; Serological Methods, a Laboratory Manual, Section IV, APS Press, St Paul, Minn.) and in Maddox, D. E. et al. (1983; J. Exp. Med. 158:1211-1216).

A wide variety of labels and conjugation techniques are known by those skilled in the art and may be used in various nucleic acid and amino acid assays. Means for producing labelled hybridisation or PCR probes for detecting sequences related to polynucleotides encoding GPR54 include oligolabeling, nick translation, end-labeling, or PCR amplification using a labelled nucleotide. Alternatively, the sequences encoding GPR54, or any fragments thereof, may be cloned into a vector for the production of an mRNA probe. Such vectors are known in the art, are commercially available, and may be used to synthesize RNA probes in vitro by addition of an appropriate RNA polymerase such as T7, T3, or SP6 and labelled nucleotides. These procedures may be conducted using a variety of commercially available kits, such as those provided by Pharmacia & Upjohn (Kalamazoo, Mich.), Promega (Madison, Wis.), and U.S. Biochemical Corp. (Cleveland, Ohio). Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding GPR54 may be cultured under conditions suitable for the expression and recovery of the protein from cell culture. The protein produced by a transformed cell may be located in the cell membrane, secreted or contained intracellularly depending on the sequence and/or the vector used. As will be understood by those of skill in the art, expression vectors containing polynucleotides which encode GPR54 may be designed to contain signal sequences which direct secretion of GPR54 through a prokaryotic or eukaryotic cell membrane. Other constructions may be used to join sequences encoding GPR54 to nucleotide sequences encoding a polypeptide domain which will facilitate purification of soluble proteins. Such purification facilitating domains include, but are not limited to, metal chelating peptides such as histidine-tryptophan modules that allow purification on immobilised metals, protein A domains that allow purification on immobilised immunoglobulin, and the domain utilised in the FLAGS extension/affinity purification system (Immunex Corp., Seattle, Wash.). The inclusion of cleavable linker sequences, such as those specific for Factor XA or enterokinase (Invitrogen, San Diego, Calif.), between the purification domain and the GPR54 GPCR encoding sequence may be used to facilitate purification. One such expression vector provides for expression of a fusion protein containing GPR54 and a nucleic acid encoding 6 histidine residues preceding a thioredoxin or an enterokinase cleavage site. The histidine residues facilitate purification on immobilised metal ion affinity chromatography (IMIAC; described in Porath, J. et al. (1992) Prot. Exp. Purif. 3: 263-281), while the enterokinase cleavage site provides a means for purifying GPR54 from the fusion protein. A discussion of vectors which contain fusion proteins is provided in Kroll, D. J. et al. (1993; DNA Cell Biol. 12:441-453).

Fragments of GPR54 may be produced not only by recombinant production, but also by direct peptide synthesis using solid-phase techniques. (Merrifield J. (1963) J. Am. Chem. Soc. 85:2149-2154.) Protein synthesis may be performed by manual techniques or by automation. Automated synthesis may be achieved, for example, using the Applied Biosystems 431A peptide synthesiser (Perkin Elmer). Various fragments of GPR54 may be synthesised separately and then combined to produce the full length molecule.

### TRANSGENIC ANIMALS

### Knockouts

In a further aspect of the present invention, there is provided a non-human GPR54 knockout mammal comprising one or more cells in which GPR54 polypeptide is functionally inactivated.

The GPR54 knockouts of the present invention may arise as a result of functional disruption of the GPR54 gene or any portion of that gene, including one or more loss of function mutations, including a deletion or replacement, of the GPR54 gene. The mutations include single point mutations, and may target coding or non-coding regions of GPR54.

Preferably, such a non-human knockout animal is a pig, a sheep or a rodent. Most preferably the non-human knockout animal is a mouse or a rat. Such non-human knockout animals may be used in screening procedures to identify agonists and/or antagonists of GPR54, as well as to test for their efficacy as treatments for diseases *in vivo*.

For example, non-human knockout animals that have been engineered to be deficient in the production of GPR54 may be used in assays to identify agonists and/or antagonists of GPR54. One assay is designed to evaluate a potential drug (a candidate ligand or compound) to determine if it produces a physiological response in the absence of GPR54 receptors. This may be accomplished by administering the drug to a non-human knockout animal as discussed above, and then assaying the non-human animal for a particular response. Any physiological parameter could be measured in this assay.

Tissues derived from the non-human GPR54 knockout animals may be used in receptor binding assays to determine whether the potential drug (a candidate ligand or compound) binds to the GPR54 receptor. Such assays can be conducted by obtaining a first receptor preparation from the non-human knockout animal engineered to be deficient in GPR54 receptor production and a second receptor preparation from a source known to bind any identified GPR54 ligands or compounds. In general, the first and second receptor preparations will be similar in all respects except for the source from which they are obtained. For example, if brain tissue from a non-human knockout animal (such as described above and below) is used in an assay, comparable brain tissue from a normal (wild type) non-human animal is used as the source of the second receptor preparation. Each of the receptor preparations is incubated with a ligand known to bind to GPR54 receptors, both alone and in the presence of the candidate ligand or compound. Preferably, the candidate ligand or compound will be examined at several different concentrations.

The extent to which binding by the known ligand is displaced by the test compound is determined for both the first and second receptor preparations. Tissues derived from knockout animals may be used in assays directly or the tissues may be processed to isolate membranes or membrane proteins, which are themselves used in the assays. A preferred knockout animal is the mouse. The ligand may be labelled using any means compatible with binding assays. This would include, without limitation, radioactive, enzymatic, fluorescent or chemiluminescent labeling (as well as other labelling techniques as described in further detail above).

Furthermore, antagonists of GPR54 receptor may be identified by administering candidate compounds, etc, to wild type animals expressing functional GPR54, and animals identified which exhibit any of the phenotypic characteristics associated with reduced or abolished expression of GPR54 receptor function.

Detailed methods for generating non-human knockout animals are described in further detail below. Transgenic gene constructs can be introduced into the germ line of an animal to make a knockout mammal. For example, one or several copies of the construct may be incorporated into the genome of a non-human mammalian embryo by standard transgenic techniques.

In an exemplary embodiment, the knockout non-human animals of the invention are produced by introducing transgenes into the germline of the non-human animal. Non-human embryonal target cells at various developmental stages can be used to introduce transgenes. Different methods are used depending the stage of development of the embryonal target cell. The specific line(s) of any non-human animal used to practice this invention are selected for general good health, good embryo yields, good pronuclear visibility in the embryo, and good reproductive fitness. In addition, the haplotype is a significant factor.

Introduction of the transgene into the non-human embryo can be accomplished by any means known in the art such as, for example, microinjection, electroporation, or lipofection. For example, the GPR54 receptor transgene can be introduced into a non-human mammal by microinjection of the construct into the pronuclei of the non-human fertilised mammalian egg(s) to cause one or more copies of the construct to be retained in the cells of the developing non-human mammal(s). Following introduction of the transgene construct into the fertilised non-human egg, the non-human egg may be incubated in vitro for varying amounts of time, or reimplanted into the non-human surrogate host, or both. *In vitro* incubation to maturity is within the scope of this invention. One common method in to incubate the non-human embryos in vitro for about 1-7 days, depending on the species, and then reimplant them into the non-human surrogate host.

The progeny of the transgenically manipulated non-human embryos can be tested for the presence of the construct by Southern blot analysis of the segment of tissue. If one or more copies of the exogenous cloned construct remains stably integrated into the genome of such non-human knockout embryos, it is possible to establish permanent non-human knockout mammal lines carrying the transgenically added construct.

The litters of non-human knockout altered mammals can be assayed after birth for the incorporation of the construct into the genome of the offspring. Preferably, this assay is accomplished by hybridising a probe corresponding to the DNA sequence coding for the desired recombinant protein product or a segment thereof onto chromosomal material from the progeny. Those non-human mammalian progeny found to contain at least one copy of the construct in their genome are grown to maturity.

For the purposes of this invention a zygote is essentially the formation of a diploid cell which is capable of developing into a complete organism. Generally, the zygote will be comprised of an egg containing a nucleus formed, either naturally or artificially, by the fusion of two haploid nuclei from a gamete or gametes. Thus, the gamete nuclei must be ones which are naturally compatible, i.e., ones which result in a viable zygote capable of undergoing differentiation and developing into a functioning organism. Generally, a euploid zygote is preferred. If an aneuploid zygote is obtained, then the number of chromosomes should not vary by more than one with respect to the euploid number of the organism from which either gamete originated.

In addition to similar biological considerations, physical ones also govern the amount (e.g., volume) of exogenous genetic material which can be added to the nucleus of the zygote or to the genetic material which forms a part of the zygote nucleus. If no genetic material is removed, then the amount of exogenous genetic material which can be added is limited by the amount which will be absorbed without being physically disruptive. Generally, the volume of exogenous genetic material inserted will not exceed about 10 picoliters. The physical effects of addition must not be so great as to physically destroy the viability of the zygote. The biological limit of the number and variety of DNA sequences will vary depending upon the particular zygote and functions of the exogenous genetic material and will be readily apparent to one skilled in the art, because the genetic material, including the exogenous genetic material, of the resulting zygote must be biologically capable of initiating and maintaining the differentiation and development of the zygote into a functional organism.

The number of copies of the transgene constructs which are added to the zygote is dependent upon the total amount of exogenous genetic material added and will be the amount which enables the genetic transformation to occur. Theoretically only one copy is required; however, generally, numerous copies are utilised, for example, 1,000-20,000 copies of the transgene construct, in order to insure that one copy is functional. As regards the present invention, there will often be an advantage to having more than one functioning copy of each of the inserted exogenous DNA sequences to enhance the phenotypic expression of the exogenous DNA sequences.

Any technique which allows for the addition of the exogenous genetic material into nucleic genetic material can be utilised so long as it is not destructive to the cell, nuclear membrane or other existing cellular or genetic structures. The exogenous genetic material is preferentially inserted into the nucleic genetic material by microinjection. Microinjection of cells and cellular structures is known and is used in the art.

Reimplantation is accomplished using standard methods. Usually, the surrogate host is anaesthetised, and the embryos are inserted into the oviduct. The number of embryos implanted into a particular host will vary by species, but will usually be comparable to the number of off spring the species naturally produces.

Knockout offspring of the surrogate host may be screened for the presence and/or expression of the transgene by any suitable method. Screening is often accomplished by Southern blot or Northern blot analysis, using a probe that is complementary to at least a portion of the transgene. Western blot analysis using an antibody against the protein encoded by the transgene may be employed as an alternative or additional method for screening for the presence of the transgene product. Typically, DNA is prepared from tail tissue and analysed by Southern analysis or PCR for the transgene. Alternatively, the tissues or cells believed to express the transgene at the highest levels are tested for the presence and expression of the transgene using Southern analysis or PCR, although any tissues or cell types may be used for this analysis.

Alternative or additional methods for evaluating the presence of the transgene include, without limitation, suitable biochemical assays such as enzyme and/or immunological assays, histological stains for particular marker or enzyme activities, flow cytometric analysis, and the like. Analysis of the blood may also be useful to detect the presence of the transgene product in the blood, as well as to evaluate the effect of the transgene on the levels of various types of blood cells and other blood constituents.

Retroviral infection can also be used to introduce transgene into a non-human animal. The developing non-human embryo can be cultured in vitro to the blastocyst stage. During this time, the blastomeres can be targets for retroviral infection (Jaenich, R. (1976) PNAS 73:1260-1264). Efficient infection of the blastomeres is obtained by enzymatic treatment to remove the zona pellucida (Manipulating the Mouse Embryo, Hogan eds. (Cold Spring Harbor Laboratory Press, Cold Spring Harbor, 1986). The viral vector system used to introduce the transgene is typically a replication-defective retrovirus carrying the transgene (Jahner et al. (1985) PNAS 82:6927-6931; Van der Putten et al. (1985) PNAS 82:6148-6152). Transfection is easily and efficiently obtained by culturing the blastomeres on a monolayer of virus-producing cells (Van der Putten, supra; Stewart et al. (1987) EMBO J. 6:383-388). Alternatively, infection can be performed at a later stage. Virus or virus-producing cells can be injected into the blastocoele (Jahner et al. (1982) Nature 298:623-628). Most of the founders will be mosaic for the transgene since incorporation occurs only in a subset of the cells which formed the transgenic non-human animal. Further, the founder may contain various retroviral insertions of the transgene at different positions in the genome which generally will segregate in the offspring. In addition, it is also possible to introduce transgenes into the germ line by intrauterine retroviral infection of the midgestation embryo (Jahner et al. (1982) supra).

A third type of target cell for transgene introduction is the embryonal stem cell (ES). ES cells are obtained from pre-implantation embryos cultured in vitro and fused with embryos (Evans et al. (1981) Nature 292:154-156; Bradley et al. (1984) Nature 309:255-258; Gossler et al. (1986) PNAS 83: 9065-9069; and Robertson et al. (1986) Nature 322:445-448). Transgenes can be efficiently introduced into the ES cells by DNA transfection or by retrovirus-mediated transduction. Such transformed ES cells can thereafter be combined with blastocysts from a non-human animal. The ES cells thereafter colonise the embryo and contribute to the germ line of the resulting chimeric animal. For review see Jaenisch, R. (1988) Science 240:1468-1474.

The present invention also pertains to a nucleic acid construct for functionally disrupting a GPR54 gene in a host cell. The nucleic acid construct comprises: a) a non-homologous replacement portion; b) a first homology region located upstream of the non-homologous replacement portion, the first homology region having a nucleotide sequence with substantial identity to a first GPR54 gene sequence; and c) a second homology region located downstream of the non-homologous replacement portion, the second homology region having a nucleotide sequence with substantial identity to a second GPR54 gene sequence, the second GPR54 gene sequence having a location downstream of the first GPR54 gene sequence in a naturally occurring endogenous GPR54 gene. Additionally, the first and second homology regions are of sufficient length for homologous recombination between the nucleic acid construct and an endogenous GPR54 gene in a host cell when the nucleic acid molecule is introduced into the host cell. In a preferred embodiment, the non-homologous replacement portion comprises an expression reporter, preferably including lacZ and a positive selection expression cassette, preferably including a neomycin phosphotransferase gene operatively linked to a regulatory element(s).

A GPR54 GPCR deficient transgenic animal may be generated as follows:

### (a)Construction of GPR54 gene targeting vector

Murine GPR54 genomic clones are isolated from a mouse large insert PAC library obtained from HGMP (Hinxton, UK) using a probe sequence amplified from a part of the predicted murine open reading frame cDNA sequence (SEQ ID NO: 4), using standard techniques. The isolated murine GPR54 genomic clones are then restriction mapped in the region of the GPR54 gene using small oligonucleotide probes and standard techniques.

The murine genomic locus is partially sequenced to enable the design of homologous arms to clone into the targeting vector. Two regions of DNA, typically between 1 and 5kb in size, from either side of the region of the open reading frame to be deleted, called the 5' and 3' homology arms, are amplified by PCR and the fragments are cloned into the targeting vector. The position of these arms is chosen so that a homologous recombination event will functionally disrupt the GPR54 gene by deleting at least the seven trans-membrane spanning regions. A targeting vector is prepared where the deleted GPR54 sequence is replaced with non-homologous sequences composed of an endogenous gene expression reporter (a frame-independent lacZ gene) upstream of a selection cassette composed of a promoted neomycin phosphotransferase (neo) gene, arranged in the same orientation as the GPR54 gene.

### (b) Transfection and Analysis of Embryonal Stem Cells

Embryonal stem cells (Evans and Kaufman, 1981) are cultured on a neomycin resistant embryonal fibroblast feeder layer grown in Dulbecco's Modified Eagles medium supplemented with 20% Fetal Calf Serum, 10% new-born calf serum, 2 mM glutamine, non-essential amino acids, 100 µM 2-mercaptoethanol and 500 u/ml leukemia inhibitory factor. Medium is changed daily and ES cells are subcultured every three days. 5x10⁶ ES cells are transfected with 5 µg of linearized plasmid by electroporation (25 µF capacitance and 400 Volts). 24 hours following electroporation the transfected cells are cultured for 9 days in medium containing 200 µg/ml neomycin. Clones are picked into 96 well plates, replicated and expanded before being screened by PCR to identify clones in which homologous recombination had occurred between the endogenous GPR54 gene and the targeting construct. Positive clones are typically identified at a rate of 1 to 5%. These clones where expanded to allow replicas to be frozen and sufficient high quality DNA to be prepared for Southern blot confirmation of the targeting event using external 5' and 3' probes, all using standard procedures (Russ et al, 2000, Nature 2000 Mar 2; 404(6773):95-9).

### (c) Generation of GPR54 Deficient Mice

C57BL/6 female and male mice are mated and blastocysts are isolated at 3.5 days of gestation. 10-12 cells from a chosen clone are injected per blastocyst and 7-8 blastocysts are implanted in the uterus of a pseudopregnant F 1 female.

A litter of chimeric pups are born containing several high level (up to 100%) agouti males (the agouti coat colour indicates the contribution of cells descendent from the targeted clone). The male chimeras are mated with female and MF1 and 129 mice, and germline transmission is determined by the agouti coat colour and by PCR genotyping respectively.

### Other non-human transgenic animals

Also contemplated according to the present invention are non-human transgenic animals which results in the over expression of GPR54 receptor or underexpression (as compared with suitable controls) of the receptor.

The animals are useful to identify agonists and antagonists of the receptor function an also therapeutically.

### ANTIBODIES

As herein described antibodies may be employed as agonists or antagonists of GPR54 polypeptide.

For the purposes of this invention, the term "antibody", unless specified to the contrary, includes but is not limited to, polyclonal, monoclonal, chimeric, single chain, Fab fragments and fragments produced by a Fab expression library. Such fragments include fragments of whole antibodies which retain their binding activity for a target substance, Fv, F(ab') and F(ab')₂ fragments, as well as single chain antibodies (scFv), fusion proteins and other synthetic proteins which comprise the antigen-binding site of the antibody. The antibodies and fragments thereof may be humanised antibodies, for example as described in EP-A-239400. Furthermore, antibodies with fully human variable regions (or their fragments), for example, as described in US Patent Nos. 5,545,807 and 6,075,181 may also be used. Neutralizing antibodies, i.e., those which inhibit biological activity of the substance amino acid sequences, are especially preferred for diagnostics and therapeutics.

Antibodies may be produced by standard techniques, such as by immunisation or by using a phage display library.

A polypeptide or peptide of the present invention may be used to develop an antibody by known techniques. Such an antibody may be capable of binding specifically to the GPR54 protein or homologue, fragment, etc.

If polyclonal antibodies are desired, a selected non-human mammal (e.g., mouse, rabbit, goat, horse, etc.) may be immunised with an immunogenic composition comprising a polypeptide or peptide of the present invention. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG (*Bacilli Calmette-Guerin*) and *Corynebacterium parvum* are potentially useful human adjuvants which may be employed if purified the substance amino acid sequence is administered to immunologically compromised individuals for the purpose of stimulating systemic defence.

Serum from the immunised non-human animal is collected and treated according to known procedures. If serum containing polyclonal antibodies to an epitope obtainable from a polypeptide of the present invention contains antibodies to other antigens, the polyclonal antibodies can be purified by immunoaffinity chromatography. Techniques for producing and processing polyclonal antisera are known in the art. In order that such antibodies may be made, the invention also provides amino acid sequences of the invention or fragments thereof haptenised to another amino acid sequence for use as immunogens in animals or humans.

Monoclonal antibodies directed against epitopes obtainable from a polypeptide or peptide of the present invention can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against orbit epitopes can be screened for various properties; i.e., for isotype and epitope affinity.

Monoclonal antibodies may be prepared using any technique which provides for the production of antibody molecules by continuous cell lines in culture. These include, but are not limited to, the hybridoma technique originally described by Koehler and Milstein (1975 Nature 256:495-497), the trioma technique, the human B-cell hybridoma technique (Kosbor et al (1983) Immunol Today 4:72; Cote et al (1983) Proc Natl Acad Sci 80:2026-2030) and the EBV-hybridoma technique (Cole et al., Monoclonal Antibodies and Cancer Therapy, pp. 77-96, Alan R. Liss, Inc., 1985).

In addition, techniques developed for the production of "chimeric antibodies", the splicing of mouse antibody genes to human antibody genes to obtain a molecule with appropriate antigen specificity and biological activity can be used (Morrison et al (1984) Proc Natl Acad Sci 81:6851-6855; Neuberger et al (1984) Nature 312:604-608; Takeda et al (1985) Nature 314:452-454). Alternatively, techniques described for the production of single chain antibodies (US Patent No. 4,946,779) can be adapted to produce the substance specific single chain antibodies.

Antibodies, both monoclonal and polyclonal, which are directed against epitopes obtainable from a polypeptide or peptide of the present invention are particularly useful in diagnosis, and those which are neutralising are useful in passive immunotherapy. Monoclonal antibodies, in particular, may be used to raise anti-idiotype antibodies. Anti-idiotype antibodies are immunoglobulins which carry an "internal image" of the substance and/or agent against which protection is desired. Techniques for raising anti-idiotype antibodies are known in the art. These anti-idiotype antibodies may also be useful in therapy.

Antibodies may also be produced by inducing *in vivo* production in the lymphocyte population or by screening recombinant immunoglobulin libraries or panels of highly specific binding reagents as disclosed in Orlandi et al (1989, Proc Natl Acad Sci 86: 3833-3837), and Winter G and Milstein C (1991; Nature 349:293-299).

Antibody fragments which contain specific binding sites for the polypeptide or peptide may also be generated. For example, such fragments include, but are not limited to, the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed to allow rapid and easy identification of monoclonal Fab fragments with the desired specificity (Huse WD et al (1989) Science 256:1275-128 1).

Techniques for the production of single chain antibodies (U.S. Pat. No. 4,946,778) can also be adapted to produce single chain antibodies to polypeptides of this invention. Also, transgenic mice, or other organisms including other non-human mammals, may be used to express humanised antibodies.

Thus, the inventors consider that GPR54 antibodies or the compositions comprising them may be of particular therapeutic use in the treatment of neurological, reproductive hormone related, and certain other conditions. Such neurological conditions include but are not limited to any one or more of the following: Alzheimers, sensory neuropathies and epilepsy.

Such reproductive hormone related conditions consist of any one or more of the group consisting of the following: the modulation of fertility, libido and the onset of puberty, lactation, osteoporosis/osteopetrosis and menopause. In addition, GPR54 agonists or antagonist may be used in the useful for hormone replacement therapy (HRT).

Other conditions include muscle wasting diseases, pain, hormone dependent cancer and obesity.

### DIAGNOSTIC ASSAYS

In a further aspect, the present invention provides a method for the diagnosis of a disease or condition selected from the group consisting of the following: Alzheimers, sensory neuropathies and epilepsy, the modulation of fertility, libido and the onset of puberty, lactation, osteoporosis/osteopetrosis, menopause, muscle wasting diseases, pain, hormone dependent cancer and obesity comprising the steps of:
(a) selecting a sample of cells from a patient to be diagnosed,
(b) comparing the expression levels and/or functional activity of GPR54 polypeptide in those sample of cells with one or more control sample/s from healthy individuals.

This invention also relates to the use of GPR54 polynucleotides, GPR54 peptide ligand encoding polynucleotides and polypeptides (as well as homologues, variants and derivatives thereof) for use in diagnosis as diagnostic reagents or in genetic analysis. Nucleic acids complementary to or capable of hybridising to GPR54 nucleic acids (including homologues, variants and derivatives), as well as antibodies against GPR54 polypeptides and GPR54 ligand polypeptides are also useful in such assays.

Detection of a mutated form of the GPR54 gene, metastin gene or other natural ligand gene, associated with a dysfunction will provide a diagnostic tool that can add to or define a diagnosis of a disease or susceptibility to a disease which results from under-expression, over-expression or altered expression of GPR54 or its natural ligands. Individuals carrying mutations in the GPR54 gene (including control sequences) may be detected at the DNA level by a variety of techniques.

For example, DNA may be isolated from a patient and the DNA polymorphism pattern of GPR54 or metastin determined. The identified pattern is compared to controls of patients known to be suffering from a disease associated with over-, under- or abnormal expression of GPR54 or metastin. Patients expressing a genetic polymorphism pattern associated with GPR54 or metastin associated disease may then be identified. Genetic analysis of the GPR54 or metastin gene may be conducted by any technique known in the art. For example, individuals may be screened by determining DNA sequence of a GPR54 or metastin allele, by RFLP or SNP analysis, etc. Patients may be identified as having a genetic predisposition for a disease associated with the over-, under-, or abnormal expression of GPR54 or metastin by detecting the presence of a DNA polymorphism in the gene sequence for GPR54 or metastin or any sequence controlling its expression.

Patients so identified can then be treated to prevent the occurrence of GPR54 or metastin associated disease, or more aggressively in the early stages of GPR54 or metastin associated disease to prevent the further occurrence or development of the disease. GPR54 or metastin associated diseases include Alzheimers, sensory neuropathies and epilepsy, the modulation of fertility, libido and the onset of puberty, lactation, osteoporosis/osteopetrosis, menopause, muscle wasting diseases, pain, hormone dependent cancer.

In a preferred embodiment, GPR54 or metastin associated diseases comprise any one of Alzheimers, sensory neuropathies and epilepsy, the modulation of fertility, libido and the onset of puberty, lactation, osteoporosis/osteopetrosis, menopause, muscle wasting diseases, pain, hormone dependent cancer and obesity. Advantageously, the disease or condition relates to modulation of the sex steroid hormonal axis.

The present invention further discloses a kit for the identification of a patient's genetic polymorphism pattern associated with GPR54 or metastin associated disease. The kit includes DNA sample collecting means and means for determining a genetic polymorphism pattern, which is then compared to control samples to determine a patient's susceptibility to GPR54 or metastin associated disease. Kits for diagnosis of a GPR54 or metastin associated disease comprising GPR54 or metastin polypeptide and/or an antibody against such a polypeptide (or fragment of it) are also provided.

Nucleic acids for diagnosis may be obtained from a subject's cells, such as from blood, urine, saliva, tissue biopsy or autopsy material. In a preferred embodiment, the DNA is obtained from blood cells obtained from a finger prick of the patient with the blood collected on absorbent paper. In a further preferred embodiment, the blood is collected on an AmpliCard.TM. (University of Sheffield, Department of Medicine and Pharmacology, Royal Hallamshire Hospital, Sheffield, England S10 2JF).

The DNA may be used directly for detection or may be amplified enzymatically by using PCR or other amplification techniques prior to analysis. Oligonucleotide DNA primers that target the specific polymorphic DNA region within the genes of interest may be prepared so that in the PCR reaction amplification of the target sequences is achieved. RNA or cDNA may also be used as templates in similar fashion. The amplified DNA sequences from the template DNA may then be analysed using restriction enzymes to determine the genetic polymorphisms present in the amplified sequences and thereby provide a genetic polymorphism profile of the patient. Restriction fragments lengths may be identified by gel analysis. Alternatively, or in conjunction, techniques such as SNP (single nucleotide polymorphisms) analysis may be employed.

Deletions and insertions can be detected by a change in size of the amplified product in comparison to the normal genotype. Point mutations can be identified by hybridising amplified DNA to labelled GPR54 or metastin nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers et al, Science (1985)230:1242. Sequence changes at specific locations may also be revealed by nuclease protection assays, such as RNase and S1protection or the chemical cleavage method. See Cotton et al., Proc Natl Acad Sci USA (1985) 85: 4397-4401. In another embodiment, an array of oligonucleotides probes comprising the GPR54 or metastin nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

Single strand conformation polymorphism (SSCP) may be used to detect differences in electrophoretic mobility between mutant and wild type nucleic acids (Orita et al. (1989) Proc Natl. Acad. Sci USA: 86:2766, see also Cotton (1993) Mutat Res 285:125-144; and Hayashi (1992) Genet Anal Tech Appl 9:73-79). Single-stranded DNA fragments of sample and control GPR54 or metastin nucleic acids may be denatured and allowed to renature. The secondary structure of single-stranded nucleic acids varies according to sequence, the resulting alteration in electrophoretic mobility enables the detection of even a single base change. The DNA fragments may be labelled or detected with labelled probes. The sensitivity of the assay may be enhanced by using RNA (rather than DNA), in which the secondary structure is more sensitive to a change in sequence. In a preferred embodiment, the subject method utilises heteroduplex analysis to separate double stranded heteroduplex molecules on the basis of changes in electrophoretic mobility (Keen et al. (1991) Trends Genet 7:5).

The diagnostic assays offer a process for diagnosing or determining a susceptibility to infections such as infections such as bacterial, fungal, protozoan and viral infections, particularly infections caused by HIV-1 or HIV-2; pain; cancers; diabetes, obesity; anorexia; bulimia; asthma; Parkinson's disease; thrombosis; acute heart failure; hypotension; hypertension; erectile dysfunction; urinary retention; metabolic bone diseases such as osteoporisis and osteopetrosis; angina pectoris; myocardial infarction; ulcers; asthma; allergies; rheumatoid arthritis; inflammatory bowel disease; irritable bowel syndrome benign prostatic hypertrophy; and psychotic and neurological disorders, including anxiety, schizophrenia, manic depression, delirium, dementia, severe mental retardation and dyskinesias, such as Huntington's disease or Gilles dela Tourett's syndrome through detection of mutation in the GPR54 or metastin gene by the methods described.

In a particularly preferred embodiment, the diagnostic assays are used to diagnose or determine susceptibility to Alzheimers disease, sensory neuropathies and epilepsy, the modulation of fertility, libido and the onset of puberty, lactation, osteoporosis/osteopetrosis, menopause, muscle wasting diseases, pain, hormone dependent cancer and obesity. Advantageously, the disease or condition relates to modulation of the sex steroid hormonal axis.

The presence of GPR54 or metastin polypeptides and nucleic acids may be detected in a sample. Thus, infections and diseases as listed above can be diagnosed by methods comprising determining from a sample derived from a subject an abnormally decreased or increased level of the GPR54 or metastin polypeptide or GPR54 or metastin mRNA. The sample may comprise a cell or tissue sample from an organism suffering or suspected to be suffering from a disease associated with increased, reduced or otherwise abnormal GPR54 or metastin expression, including spatial or temporal changes in level or pattern of expression. The level or pattern of expression of GPR54 or metastin in an organism suffering from or suspected to be suffering from such a disease may be usefully compared with the level or pattern of expression in a normal organism as a means of diagnosis of disease.

In general therefore, the invention includes a method of detecting the presence of a nucleic acid comprising a GPR54 or metastin nucleic acid in a sample, by contacting the sample with at least one nucleic acid probe which is specific for said nucleic acid and monitoring said sample for the presence of the nucleic acid. For example, the nucleic acid probe may specifically bind to the GPR54 or metastin nucleic acid, or a portion of it, and binding between the two detected; the presence of the complex itself may also be detected. Furthermore, the invention encompasses a method of detecting the presence of a GPR54 or metastin polypeptide by contacting a cell sample with an antibody capable of binding the polypeptide and monitoring said sample for the presence of the polypeptide. This may conveniently be achieved by monitoring the presence of a complex formed between the antibody and the polypeptide, or monitoring the binding between the polypeptide and the antibody. Methods of detecting binding between two entities are known in the art, and include FRET (fluorescence resonance energy transfer), surface plasmon resonance, etc.

Decreased or increased expression can be measured at the RNA level using any of the methods well known in the art for the quantitation ofpolynucleotides, such as, for example, PCR, RT-PCR, RNase protection, Northern blotting and other hybridisation methods. Assay techniques that can be used to determine levels of a protein, such as a GPR54 or metastin, in a sample derived from a host are well-known to those of skill in the art. Such assay methods include radioimmunoassays, competitive-binding assays, Western Blot analysis and ELISA assays.

The present invention relates to a diagnostic kit for a disease or susceptibility to a disease which comprises any one of Alzheimers, sensory neuropathies and epilepsy, the modulation of fertility, libido and the onset of puberty, lactation, osteoporosis/osteopetrosis, menopause, muscle wasting diseases, pain, hormone dependent cancer and obesity. Advantageously, the disease or condition relates to modulation of the sex steroid hormonal axis.

A particularly preferred diagnostic kit is used to detect or diagnose disease or susceptibility to any of the following: Alzheimers, sensory neuropathies and epilepsy, the modulation of fertility, libido and the onset of puberty, lactation, osteoporosis/osteopetrosis, menopause, muscle wasting diseases, pain, hormone dependent cancer and obesity. Advantageously, the disease or condition relates to modulation of the sex steroid hormonal axis.

The diagnostic kit comprises a GPR54 or metastin polynucleotide or a fragment thereof; a complementary nucleotide sequence; a GPR54 or metastin polypeptide or a fragment thereof, or an antibody to a GPR54 or metastin polypeptide.

### PROPHYLACTIC AND THERAPEUTIC METHODS

This invention provides methods of treating an abnormal conditions related to both an excess of and insufficient amounts of GPR54 polypeptide activity and consider that GPR54 polypeptide, binding proteins thereof and/or the nucleic acids encoding them may be of particular use in the treatment of neurological, reproductive hormone related, and certain other conditions. Such neurological conditions include but are not limited to any one or more of the following: Alzheimers, sensory neuropathies and epilepsy.

Such reproductive hormone related conditions consist of any one or more of the group consisting of the following: the modulation of fertility, libido and the onset of puberty, lactation, osteoporosis/osteopetrosis and menopause. In addition, GPR54 agonists or antagonist may be used in the useful for hormone replacement therapy (HRT).

Other conditions include muscle wasting diseases, pain, hormone dependent cancer and obesity.

If the activity of GPR54 is in excess, several approaches are available. One approach comprises administering to a subject an inhibitor compound (antagonist) as herein above described along with a pharmaceutically acceptable carrier in an amount effective to inhibit activation by blocking binding of ligands to the GPR54, or by inhibiting a second signal, and thereby alleviating the abnormal condition.

In another approach, soluble forms of GPR54 polypeptides still capable of binding the ligand in competition with endogenous GPR54 may be administered. Typical embodiments of such competitors comprise fragments of the GPR54 polypeptide.

In still another approach, expression of the gene encoding endogenous GPR54 polypeptide can be inhibited using expression blocking techniques. Known such techniques involve the use of antisense sequences, either internally generated or separately administered. See, for example, O'Connor, J Neurochem (1991) 56:560 in Oligodeoxynucleotides as Antisense Inhibitors of Gene Expression, CRC Press, Boca Raton, Fla. (1988). Alternatively, oligonucleotides which form triple helices with the gene can be supplied. See, for example, Lee et al., Nucleic Acids Res (1979) 6:3073; Cooney et al., Science (1988) 241:456; Dervan et al., Science (1991) 251:1360. These oligomers can be administered per se or the relevant oligomers can be expressed in vivo.

For treating abnormal conditions related to an under-expression of GPR54 and its activity, several approaches are also available. One approach comprises administering to a subject a therapeutically effective amount of a compound which mimics ligand bound GPR54, i.e., an agonist as described above, in combination with a pharmaceutically acceptable carrier, to thereby alleviate the abnormal condition. Alternatively, gene therapy may be employed to effect the endogenous production of GPR54 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells in vivo and expression of the polypeptide in vivo. For overview of gene therapy, see Chapter 20, Gene Therapy and other Molecular Genetic-based Therapeutic Approachs, (and references cited therein) in Human Molecular Genetics, T Strachan and A P Read, BIOS Scientific Publishers Ltd (1996).

### FORMULATION AND ADMINISTRATION

Peptides, such as the soluble form of GPR54 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localize, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimisation, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide ex vivo, and for example, by the use of a retroviral plasmid vector. The cells are then introduced into the subject.

### PHARMACEUTICAL COMPOSITIONS

The present invention also provides a pharmaceutical composition comprising administering a therapeutically effective amount of the GPR54 polypeptide, binding molecules thereof or the nucleic acid encoding them according to the present invention and optionally a pharmaceutically acceptable carrier, diluent or excipients (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### ADMINISTRATION

Typically, a physician will determine the actual dosage which will be most suitable for an individual subject and it will vary with the age, weight and response of the particular patient. The dosages below are exemplary of the average case. There can, of course, be individual instances where higher or lower dosage ranges are merited.

The pharmaceutical compositions of the present invention may be administered by direct injection. The composition may be formulated for parenteral, mucosal, intramuscular, intravenous, subcutaneous, intraocular or transdermal administration. Typically, each protein may be administered at a dose of from 0.01 to 30 mg/kg body weight, preferably from 0.1 to 10 mg/kg, more preferably from 0.1 to 1 mg/kg body weight.

The term "administered" includes delivery by viral or non-viral techniques. Viral delivery mechanisms include but are not limited to adenoviral vectors, adeno-associated viral (AAV) vectors, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof The routes for such delivery mechanisms include but are not limited to mucosal, nasal, oral, parenteral, gastrointestinal, topical, or sublingual routes.

The term "administered" includes but is not limited to delivery by a mucosal route, for example, as a nasal spray or aerosol for inhalation or as an ingestable solution; a parenteral route where delivery is by an injectable form, such as, for example, an intravenous, intramuscular or subcutaneous route.

The term "co-administered" means that the site and time of administration of each of for example, the polypeptide of the present invention and an additional entity such as adjuvant are such that the necessary modulation of the immune system is achieved. Thus, whilst the polypeptide and the adjuvant may be administered at the same moment in time and at the same site, there may be advantages in administering the polypeptide at a different time and to a different site from the adjuvant. The polypeptide and adjuvant may even be delivered in the same delivery vehicle - and the polypeptide and the antigen may be coupled and/or uncoupled and/or genetically coupled and/or uncoupled.

The polypeptide, polynucleotide, peptide, nucleotide, and optionally an adjuvant may be administered separately or co-administered to the host subject as a single dose or in multiple doses.

The pharmaceutical compositions of the present invention may be administered by a number of different routes such as injection (which includes parenteral, subcutaneous and intramuscular injection) intranasal, mucosal, oral, intra-vaginal, urethral or ocular administration.

The pharmaceutical compositions of the present invention may be conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, may be 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer.

The invention will now be described with particular reference to the examples which should not be considered limiting of the invention.

### EXAMPLES

### Example 1. Transgenic GPR54 Knock-Out Mouse

### Construction of GPR54 Gene Targeting Vector

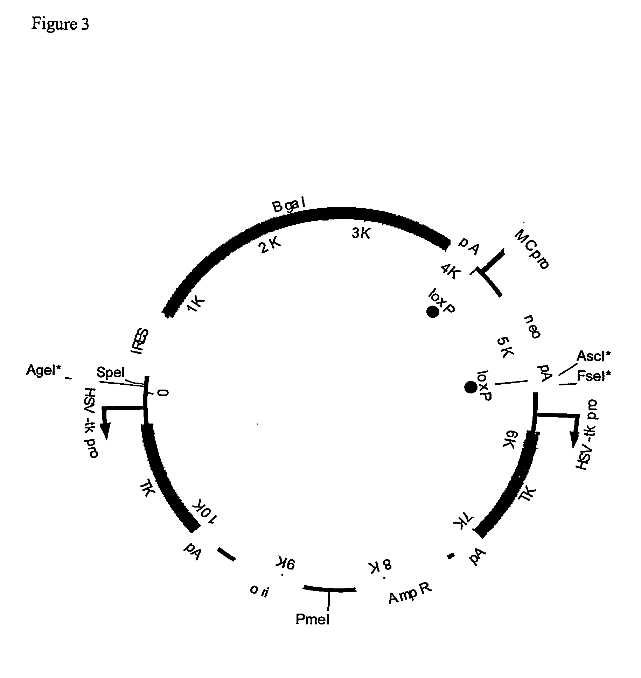

The murine GPR54 gene was identified bioinformatically, and was found to be situated within a 10.3kb genomic contig, derived from a PAC library. Further bioinformatic work extended this contig to 28kb. This contig provided sufficient flanking sequence information to enable the design of homologous arms to clone into the targeting vector (the structure of the targeting vector used, including the relevant restriction sites, is shown in Figure 3).

The murine GPR54 gene has five coding exons. The targeting strategy is designed to remove part of the first exon, prior to the start of the 7tm coding domains, and the majority of the second 7tm-containing exon. A 4.7 kb 5' homologous arm and a 1.0 kb 3' homologous arm flanking the 7tm-containing exons to be deleted are amplified by PCR and the fragments are cloned into the targeting vector. The 5' end of each oligonucleotide primer used to amplify the arms is synthesised to contain a different recognition site for a rare-cutting restriction enzyme, compatible with the cloning sites of the vector polylinkers and absent from the arms themselves. In the case of GPR54, the primers are designed as listed in the sequence table below, with 5' arm cloning enzymes of AgeI/SpeI and 3' arm cloning enzymes of AscI/FseI.

In addition to the arm primer pairs (5'arm5'1 (AgeI) / 5'arm3'(SpeI) and 3'arm5'end AscI /3'arm3'2 Fse), further primers specific to the GPR54 locus are designed for the following purposes: 5' and 3' probe primer pairs (5'probeFII/5'probeRII and 3'probeF1/3'probeRl) to amplify two short 150-300bp fragments of non-repetitive genomic DNA external to and extending beyond each arm, to allow Southern analysis of the targeted locus, in isolated putative targeted clones; a mouse genotyping primer pair (hetF and hetR) which allows differentiation between wild-type, heterozygote and homozygous mice, when used in a multiplex PCR with a vector specific primer, in this case, Asc403; and lastly, a target screening primer (3P3A) which anneals upstream of the end of the 3' arm region, and which produces a target event specific 1.2kb amplimer when paired with a primer specific to the 3' end of the vector (neo36). This amplimer can only be derived from template DNA from cells where the desired genomic alteration has occurred and allows the identification of correctly targeted cells from the background of clones containing randomly integrated copies of the vector. The location of these primers and the genomic structure of the GPR54 locus used in the targeting strategy is shown in SEQ ID NO: 10.

**Table 1. GPR54 Primer Sequences**

| | |
|---|---|
| musHarryP 5'probeF II | GTGTACCAGGTGAGGAGGCCATCAGAGGTG |
| musHarryP5'probeRII | TGTCATCCTGAGGCCCAATGGTTCTTCAGG |
| musHarry5'arm5'1 Age | AAAACCGGT AAATGCTGTTAATCCTGCCAAGAG |
| musHarry5'arm3' Spe | ATAACTAGTGTAGCGAAAAACAGGGGAAC |
| musHarry3'arm5'end AscI | AAATTCGTCAACTACATCCAGC |
| musHarry3'arm3' 2 Fse | GGGAAGTGGGATAGACACG |
| musHarry 3P3A | GGAAAAGCTAAGAACTAAGTGTGG |
| musHarry3'probeF1 | ATGAGTGTGGACCGCTGGTATGTGAC |
| musHarry3'probeR1 | TCTGAGACTGAGTATGTGCCCTTG |
| musHarryP heft | TCACTCGGACCCGGATGTACAGGTCAG |
| musHarryP hetR | AGCCCGCGTACCTGCTGGATGTAGTTG |
| Asc403 | CAGCCGAACTGTTCGCCAGGCTCAAGG |
| Neo36 | CGCATCGCCTTCTATCGCCTTCTTGAC |

The position of the homology arms is chosen to functionally disrupt the GPR54 gene by deleting the most 5' of the seven transmembrane spanning regions. A targeting vector is prepared where the deleted GPR54 sequence is replaced with non-homologous sequences composed of an endogenous gene expression reporter (a frame independent lacZ gene) upstream of a selection cassette composed of a promoted neomycin phosphotransferase (neo) gene arranged in the same orientation as the GPR54 gene.

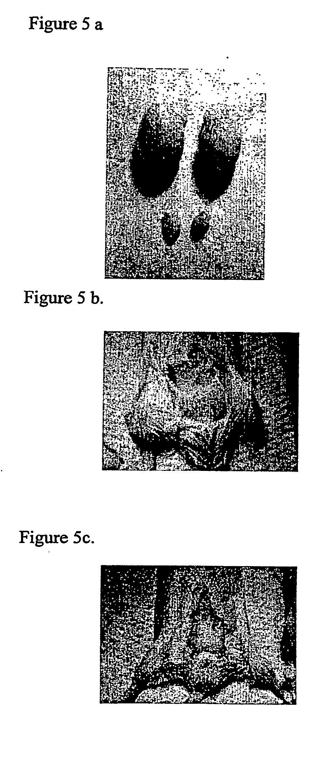

Once the 5' and 3' homology arms had been cloned into the targeting vector pTK4IBLMNL (see Figure 5), a large highly pure DNA preparation is made using standard molecular biology techniques. 20 µg of the freshly prepared endotoxin free DNA is restricted with another rare-cutting restriction enzyme PmeI, present at a unique site in the vector backbone between the ampicillin resistance gene and the bacterial origin of replication. The linearized DNA is then precipitated and resuspended in 100 µl of Phosphate Buffered Saline, ready for electroporation.

24 hours following electroporation the transfected cells are cultured for 9 days in medium containing 200 g/ml neomycin. Clones are picked into 96 well plates, replicated and expanded before being screened by PCR (using primers 3P3A and neo36, as described above) to identify clones in which homologous recombination had occurred between the endogenous GPR54 gene and the targeting construct. Positive clones can be identified at a rate of 1 to 5%. These clones are expanded to allow replicas to be frozen and sufficient high quality DNA to be prepared for Southern blot confirmation of the targeting event using the external 5' and 3' probes prepared as described above, all using standard procedures (Russ et al, Nature 2000 Mar 2;404(6773):95-92000). When Southern blots of DNA digested with diagnostic restriction enzymes are hybridised with an external probe, homologously targeted ES cell clones are verified by the presence of a mutant band as well an unaltered wild-type band. For instance, using the 5' probe, BglII digested DNA will give a 9kb wild-type band, and a 14.5kb targeted band; HindIII will give a 15kb wild-type band, and a 9.5kb targeted band; and XbaI will give a 15kb wild-type band and a 19.5kb targeted band. Similarly, using the 3' probe, BamHI will give a 7.5kb wild-type band, and a 4kb targeted band; and EcoRI will give a 7kb wild-type band, and a 4.5kb targeted band.

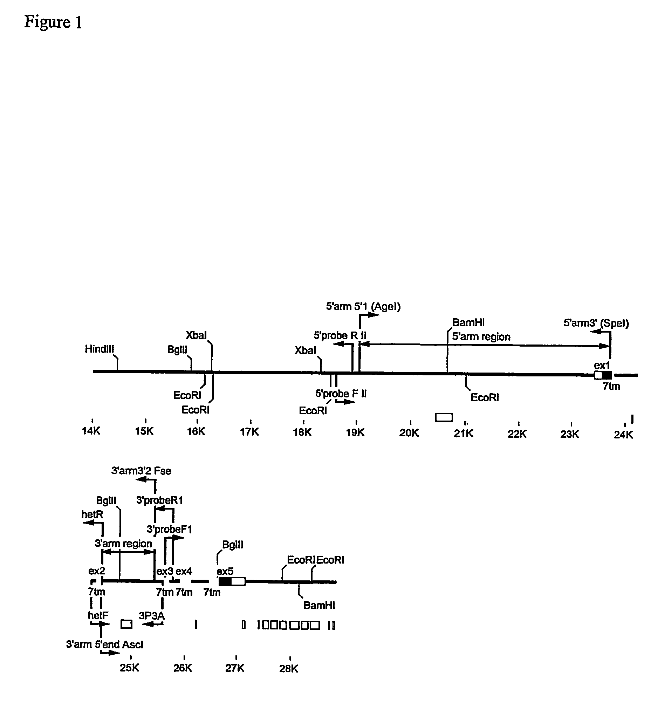

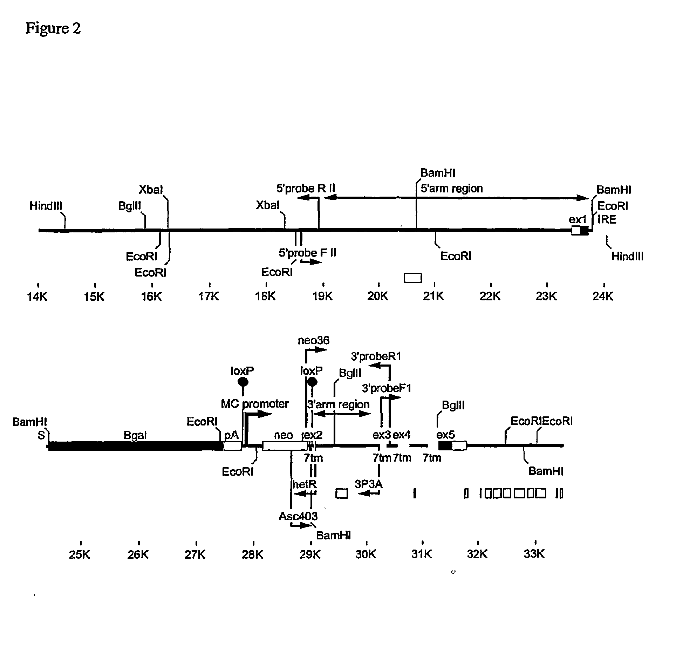

The structure of the genomic locus of mouse GPR54 before knock-out is depicted in Figure 1. The structure of the genomic locus of mouse GPR54 after knock-out is depicted in Figure 2. The sites for the enzymes relevant to the Southern verification have been annotated.

### Generation of GPR54 GPCR Deficient Mice

C57BL/6 female and male mice are mated and blastocysts are isolated at 3.5 days of gestation. 10-12 cells from a chosen clone are injected per blastocyst and 7-8 blastocysts are implanted in the uterus of a pseudopregnant F1 female. A litter of chimeric pups are born containing several high level (up to 100%) agouti males (the agouti coat colour indicates the contribution of cells descendent from the targeted clone). These male chimeras are mated with female MF1 and 129 mice, and germline transmission is determined by the agouti coat colour and by PCR genotyping respectively.

PCR Genotyping is carried out on lysed tail clips, using the primers hetF and hetR with a third, vector specific primer (Asc403). This multiplex PCR allows amplification from the wild-type locus (if present) from primers hetF and hetR giving a 217 bp band. The site for hetF is deleted in the knockout mice, so this amplification will fail from a targeted allele. However, the Asc403 primer will amplify a 439 bp band from the targeted locus, in combination with the hetR primer which anneals to a region just inside the 3' arm. Therefore, this multiplex PCR reveals the genotype of the litters as follows: wild-type samples will exhibit a single 217 bp band; heterozygous DNA samples yield two bands at 217 bp and 439 bp; and the homozygous samples will show only the target specific 439 bp band.

### Example 2

### B)Results

### I) Gene expression patterns

### 1) electronic Northern

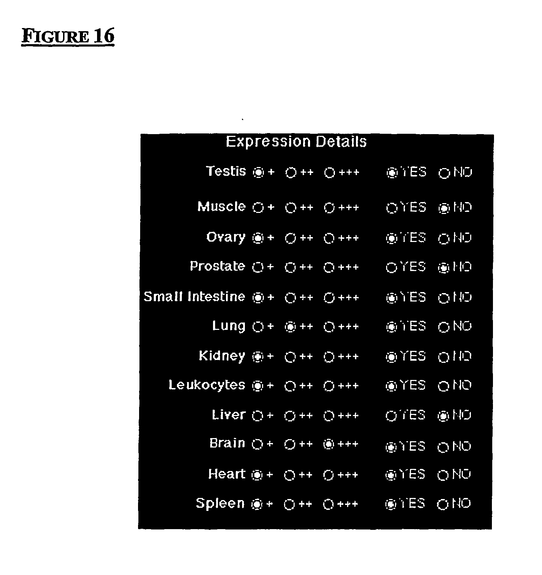

An electronic Northern is shown in Figure 16.

PCR results. Expression in a given tissues is stated as (-) not detected, (+) detected low level abundance, (++) detected medium level abundance, (+++) detected high level abundance. Testis +, Muscle -, Ovary +, Prostate -, Small intestine +, Lung ++, Kidney +, Leukocytes +, Liver -, Brain +++, Heart +, Spleen +

### Results of Est database searches

| | | |
|---|---|---|
| BF470621 | Mouse | Normalised libraries from ten |
| BE309856 | Mouse | Mammary tumour, gross tissue |
| AL541044 | Human | Placenta |
| BF470621 | Mouse | Brain (pooled) |
| BB193083 | Mouse | Spinal cord |
| AI823800 | Human | Kidney tumour |
| AA887801 | Human | colon tumour |

### 2) List of Lac Z stained structures

The following structures contained evidence of *lacZ* expression in the *lacZ* assay: brain (see sub regions below), spinal cord (sensory areas), testis.

LacZ expression was found in discrete areas of the brain, such as hippocampus (strongest area), the suprachiasmatic nucleus, the mammillary body, the pons and the cerebellum, as well as in the dorsal (sensory) part of the spinal cord.

### Observations of microtome sections

6 Wildtype and 6 mutant GPR54 knockout mouse brains were fixed and 40 micrometre sections cut on a freezing microtome.

Observation of sections under the microscope revealed localisation of LacZ staining in the following areas:
Hippocampus
Olfactory bulbs
Preoptic hypothalamus
Periventricular hypothalamus
Habenular nucleus
Hypothalamic tract
Cochlear nucleus
Supramamillary body

### 3) Lac Z Expression pictures

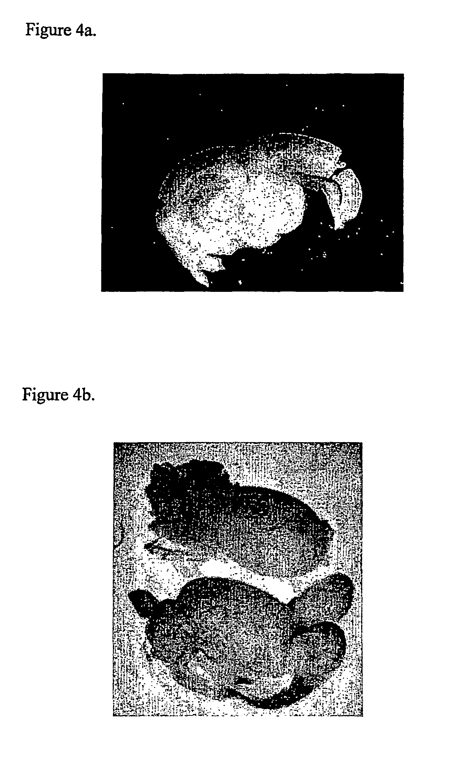

See Figure 4a Mutant brain slice and Figure 4b heterozygote and wild type testes.

### II) Anatomical observations

Harry Potter knockout mice display some gross anatomical abnormalities: mutants have a general strong atrophy of reproductive tract (atrophied preputial glands, micropenis, very small testicles and seminal vesicles/coagulatory glands in males), a reduced muscle mass, reduced brown fat mass, a smaller stomach and sub-maxillary salivary glands.

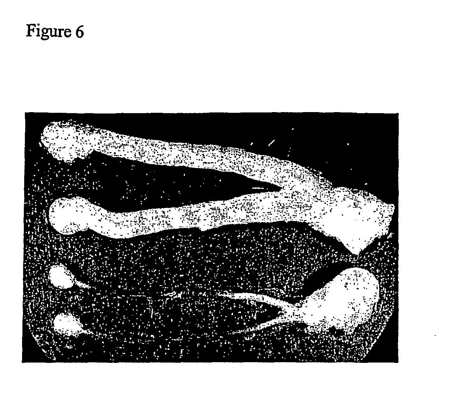

Female mutants have underdeveloped mammary glands, atrophied ovaries (Figure 6) uterus and oviducts.

The anogenital distance is also significantly shorter in male mutants as compared to wildtypes (in cm: mutants: 0.97 ± 0.03 wildtypes 1.4 ± 0.3, p<0.001), so much so that this has lead to incorrect sexing of the animals by caring staff. No difference is observed in females, however.

### Uterus and ovaries (top, wild type; bottom, mutant)

### III) Behaviour

All animals were housed with free access to food and water under a light-dark cycle of 12h light/12h darkness with lights on at 7am.

Mice were tested at 8 to 10 weeks of age, in the morning between 10h and 12h for all experiments except the Barnes maze and sexual behaviour which were done in the afternoon between 15h and 17h.

### Tests showing difference between mutants and wiltypes

### a) Barnes maze

A test for spatial memory, the Barnes maze is a white circular platform designed to require the discrimination of a particular place. It is composed of a circular platform, with 18 circular holes evenly spaced around the circumference. Underneath one of the hole is a black escape box. The Barnes maze takes advantage of the natural tendency of rodents to avoid brightly lit unenclosed surfaces and seek darkened enclosed shelter.

The animals are trained over 10 days and the latency to enter the escape box as well as the number of errors and the search strategy are recorded.

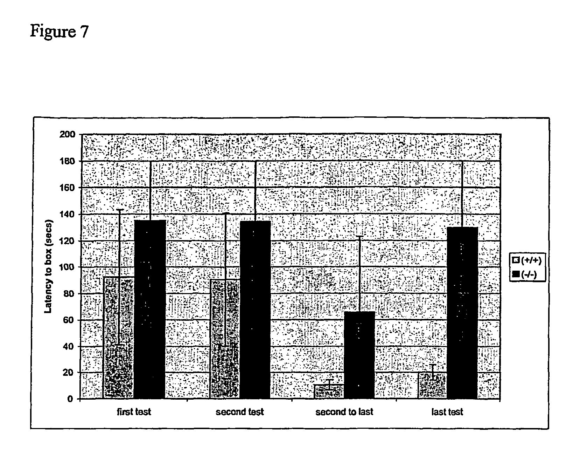

There is a trend towards lower performance on male mutants on this test (Figure 7)

### b) Mating behaviour

5 male mutants and 5 wiltype littermates were placed in a new cage with a wildtype female in estrus (assessed by vaginal smear) and observed for 30 minutes. Both displayed interest (sniffing) towards the females and mated within the observation period.

The mutants displayed no interest towards the females and did not mate within the observation period. In addition, several male and female mutant mice have been sharing cages without any pregnancy, suggesting that the GPR54 knockout mice are sterile.

### c) estrus cycle

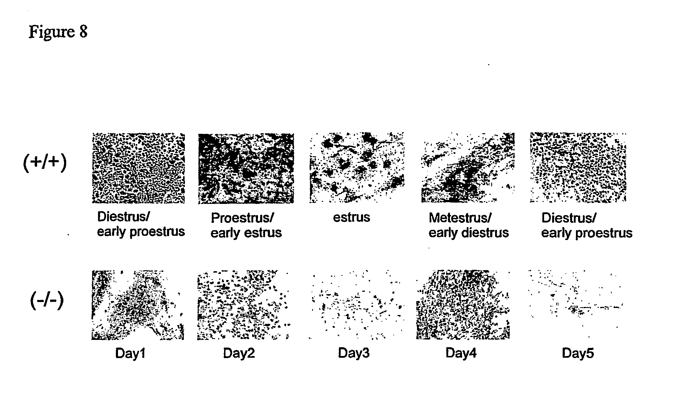

6 mutant females and 6 wildtype littermates were submitted to a smear test during 5 consecutive days. Four 3 weeks old +/+ females were also tested. The slides were stained using methylene blue. All wiltypes displayed clear estrus cycle stages, but none of the mutants did. The vaginal smears of the -/- females resembled that of the 3 weeks old wildtypes (Figure 8)

### d) Physiology

15 mating pairs comprising GPR54 mutants of either sex with wildtype mice of the opposite sex were set up, but never produced a litter. GPR54 mutants, both male and female, are therefore sterile.

### 1) Weights

### a) Organs weights

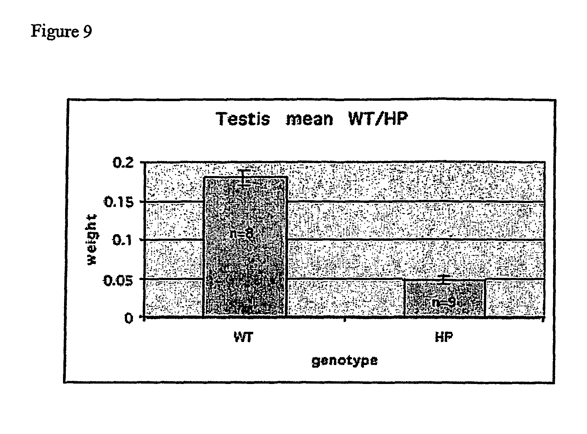

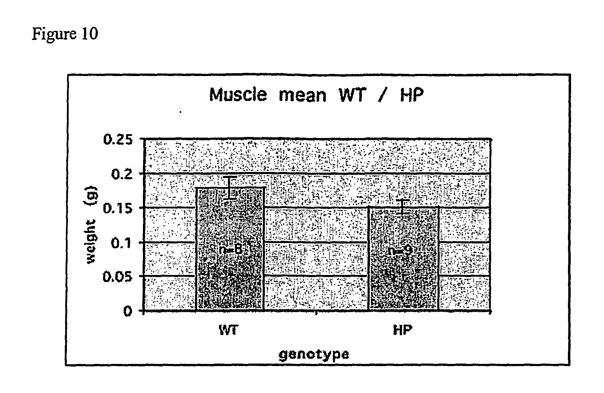

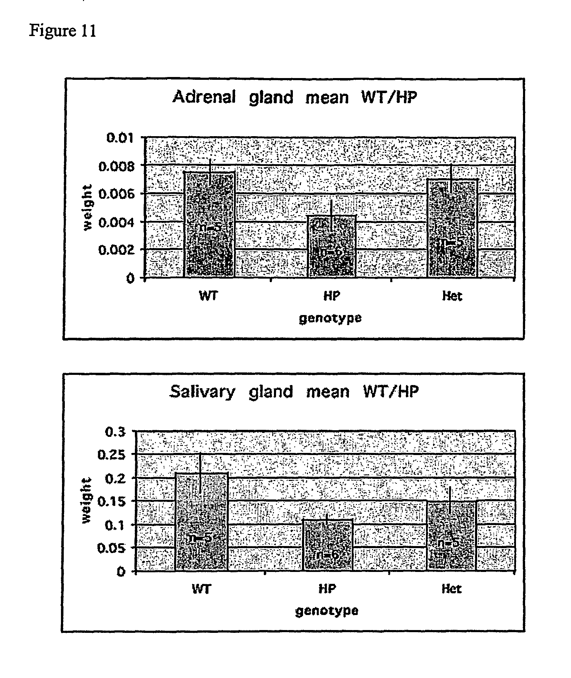

The weight of the testes (Figure 9), muscle (Figure 10), adrenal gland (Figure 11a) and salivary gland (Figure 11b) were measured.

### 2) Assays

### a) Testosterone, Oestrogen and GnRH assay

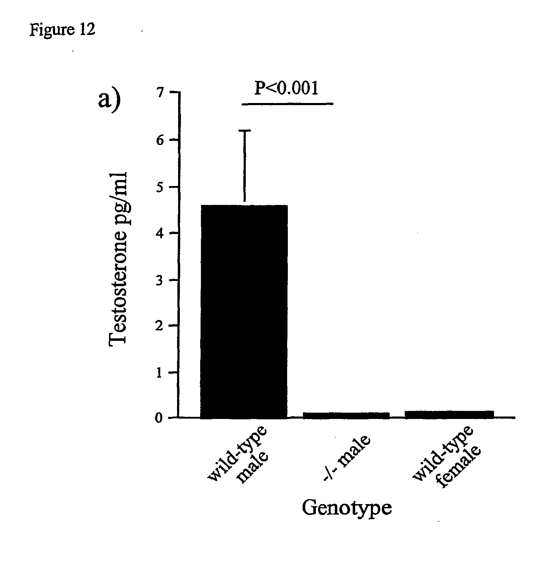

Testosterone was measured in 6 male mutants and 6 wildtype mice. Mutants have significantly lower levels of testosterone (P<0.05) compared with wildtype mice (Figure 12).

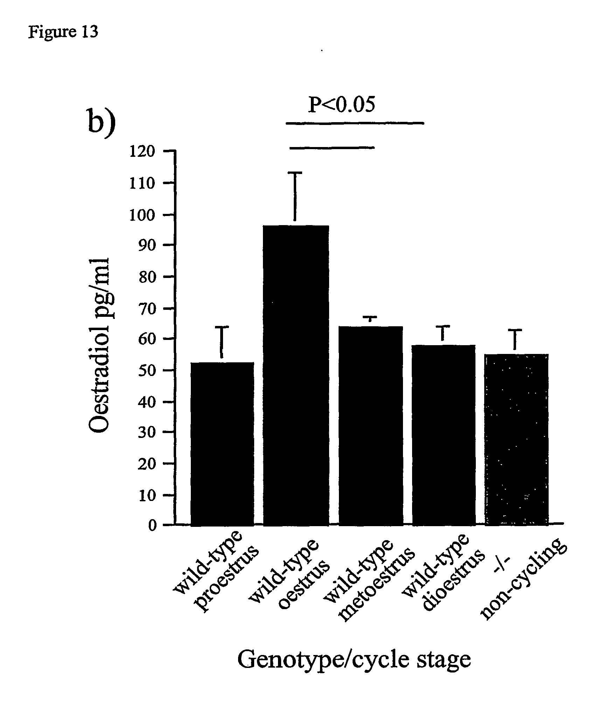

Mutant females had significantly lower levels of oestrogen compared with normal females at oestrus consistent with lack of an oestrus cycle. Wild-type oestradiol levels measured at proestrus, oestrus, metoestrus, and dioestrus shows normal cycling levels (Figure 13).

Levels of GnRH in the hypothalamus were shown to be comparable to wild type animals. This showed that the mutation had no effect on the synthesis of GnRH in knockouts (not shown).

### b) LH/FSH Assay

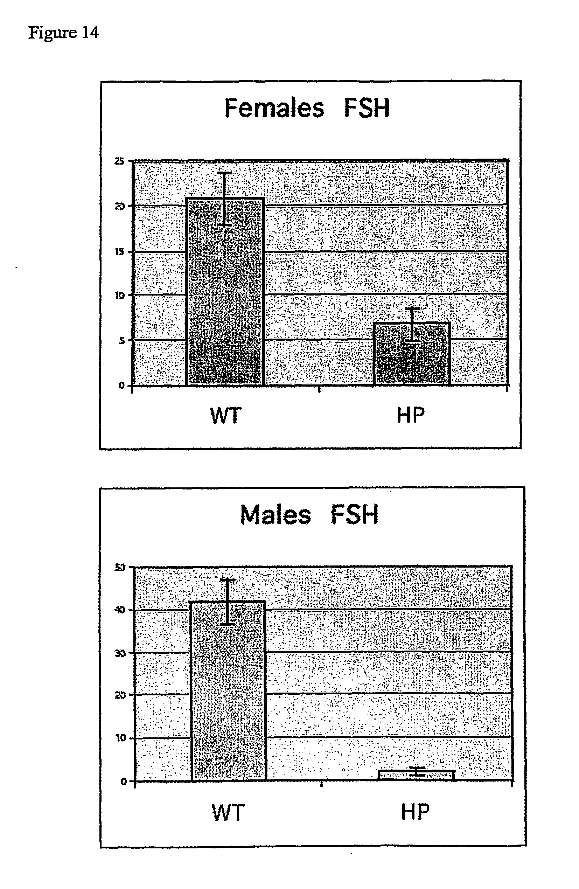

FSH is much lower in mutants (Figure 14 a females and Figure 14b males). There is no difference in LH levels (not shown).

### c) Exogenous Gonadotrophins.

Administration of 1000iu of PMS (pregnant mare serum - FSH-like gonadotrophins) followed two days later by 1000iu ofbHCG (beta human chorionic gonadotrophins) produced ovulation of mature oocytes in 50% of mutant females (n=6), indicating that end organs retain the capacity to respond to natural gonadotrophins. This is consistent with the hypothesis that GPR54 receptors and ligands are acting at the level of the pituitary hypothalamic axis and not the end-organs.

### 3) Administration of exogenous GnRH and effects on FSH and LH secretion from the pituitary

Method: 25ng of GnRH peptide was injected 4 times in 30 min intervals and the animals killed to collect blood and pituitary 30min after last injection. All mice were killed between 11 am-1pm.

Animal groups: 6 WT injected with just PBS (control), 6 WT injected with GnRH made in PBS and 6 HP injected with GnRH/PBS. All WT animals were staged and used at dioestrous (end of LH and FSH surge). All mice aged 2-4 months. This was repeated for LH and FSH measurements.

Serum FSH assay: There is a 1.9-fold increase in FSH in serum of WT injected with GnRH compared to those injected with just PBS. The HP level shows a 1.7-fold increase against WT injected with just PBS, indicating the mutant animals remain at least partially responsive to GnRH. This suggests the action of GPR54 may be in the control of GnRH release in the hypothalamus.

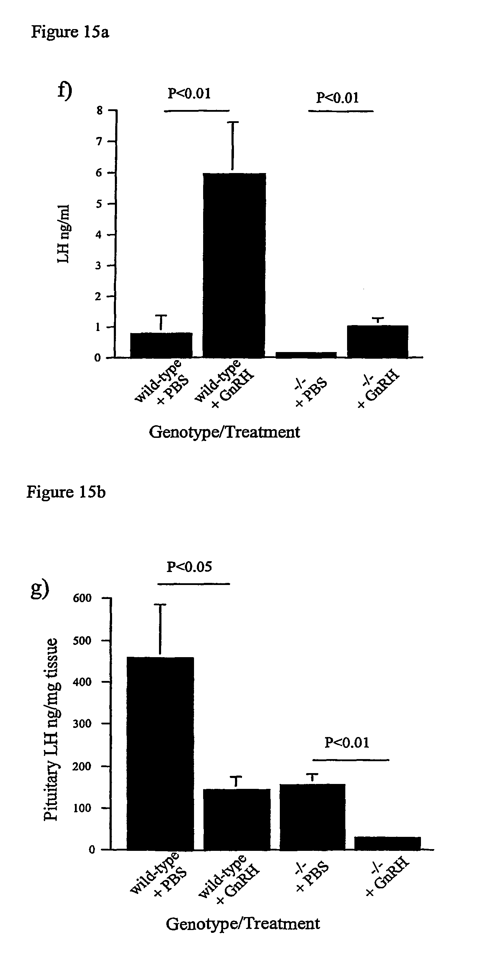

Serum LH assay: There is a 5-fold increase in LH in serum of WT injected with GnRH compared to those injected with just PBS. The HP level shows a 5-fold increase against WT injected with just PBS. This showed that the release of LH is stimulated by exogenous GnRH (Figure 15a).

Measurement of the depletion of LH levels in the pituitary after stimulation by exogenous was measured. The result showed a corresponding depletion of LH levels in the pituitary showing that the mutants still responded to GnRH and LH was released from the pituitary (Figure 15b). There were no deleterious effects of knocking out the gene in the mutants.

## Claims

1. A method of identifying a molecule suitable for the treatment of a reproductive hormone related disorder, for use as a hormone replacement therapy (HRT), for manipulating the sex hormone axis of an animal or for modulation of fertility and libido, the method comprising determining if a candidate molecule is an agonist or antagonist of a GPR54 polypeptide.

2. Use of a GPR54 polypeptide or polynucleotide for the identification of a molecule suitable for the treatment of a reproductive hormone related disorder, for use as a hormone replacement therapy (HRT), for manipulating the sex hormone axis of an animal or for modulation of fertility and libido.

3. A method of identifying an antagonist of a GPR54 polypeptide, the method comprising administering a candidate molecule to a non-human animal and determining whether the non-human animal exhibits a phenotype selected from the group consisting of: alterations in reproductive organ and associated organ morphology, including any of the following: atrophy of preputial glands, micropenis, reduced testicle size, reduced seminal vesicle size, reduced coagulatory gland size, reduced muscle mass, reduced brown fat mass, reduced stomach size, reduced sub-maxillary salivary glands, reduced anogenital distance; alterations in reproductive hormone levels and/or patterns (including cycles), including any of the following: reduced testosterone levels, reduced oestrogen levels, reduced gonadotrophin levels, reduced follicle stimulating hormone (FSH) levels; alterations in sexual/reproductive behaviour, including lack of sexual interest and lack of mating; and sterility.

4. A method according to claim 3, in which the non-human animal expresses functional GPR54 polypeptide.

5. A method according to claim 3 or 4, in which the non-human animal is a wild type non-human animal.

6. A method according to claim 3, 4 or 5, in which the non-human animal is a rodent, preferably a mouse.

7. A method for providing an indication useful in the diagnosis of or a determination of susceptibility to a reproductive hormone related disorder in an individual, the method comprising detecting a change in the expression pattern or level of a GPR54 polypeptide in a sample from the individual.

8. A method for providing an indication useful in the diagnosis of or a determination of susceptibility to a reproductive hormone related disorder in an individual, the method comprising detecting a polymorphism in a GRP54 polynucleotide in a sample from the individual.

9. A method according to any of claims 1 and 3-8, in which the agonist or antagonist comprises an immunoglobulin, preferably capable of binding specifically to a GPR54 polypeptide.

10. Use of a transgenic non-human animal having a functionally disrupted endogenous GPR54 gene or an isolated cell or tissue thereof, in a method of identifying an agonist or antagonist of a GPR54 polypeptide for the treatment of a reproductive hormone related disorder, for use as a hormone replacement therapy (HRT), for manipulating the sex hormone axis of an animal or for modulation of fertility and libido.

11. Use according to claim 10, in which the transgenic non-human animal is a rodent, preferably a mouse.

12. Use of a transgenic non-human animal defined in any of claims 10 or 11, or an isolated cell or tissue thereof, as a model for a reproductive disorder or a reproductive hormone related disorder.

13. A method or use according to any preceding claim, in which the reproductive hormone related disorder is selected from the group consisting of: a disorder of fertility and a disorder of libido, modulation of fertility, contraception, libido and the onset of puberty, lactation, osteoporosis/osteopetrosis, menopause including a hormone imbalance related to the menopause, hormone dependent cancer and benign prostatic hypertophy.

## Patentansprüche

1. Verfahren zum Auffinden eines Moleküls, das für die Behandlung von auf Sexualhormone bezogenen Störungen geeignet ist, zur Verwendung als Hormonersatztherapie (HRT), zur Beeinflussung der sexualhormonalen Achse eines Tieres oder zur Regulierung der Fruchtbarkeit und der Libido, umfassend die Feststellung, ob ein Kandidatenmolekül ein Agonist oder ein Antagonist eines GPR54-Polypeptids ist.

2. Verwendung eines GPR54-Polypeptids oder -Polynukleotids zum Auffinden eines Moleküls, das für die Behandlung von auf Sexualhormone bezogenen Störungen geeignet ist, zur Verwendung als Hormonersatztherapie (HRT), zur Beeinflussung der sexualhormonalen Achse eines Tieres oder zur Regulierung der Fruchtbarkeit und der Libido.

3. Verfahren zum Auffinden eines Antagonisten eines GPR54-Polypeptids, umfassend die Verabreichung eines Kandidatenmoleküls an ein nichtmenschliches Tier und die Feststellung, ob das nichtmenschliche Tier ein Phänotyp-Merkmal zeigt, das aus der aus Veränderungen in der Morphologie der Fortpflanzungsorgane oder der zugehörigen Organe, einschließlich Atrophie der Präputialdrüsen, Mikropenis, verminderte Hodengröße, verminderte Größe der Samenbläschen, verminderte Größe der Koagulationsdrüse, verminderte Muskelmasse, verringerte braune Fettmasse, verkleinerter Magen, verkleinerte submaxilläre Speicheldrüsen, verminderter Anogenitalabstand; Veränderungen der Sexualhormonspiegel und/oder -muster (einschließlich der Zyklen), einschließlich verminderter Testosteronspiegel, verminderter Östrogenspiegel, verminderter Gonadotropinspiegel, verminderter Follikelhormon(FSH)spiegel; Veränderungen im Sexual-/Fortpflanzungsverhalten, einschließlich Fehlen sexuellen Interesses und fehlendes Paaren; und Sterilität bestehenden Gruppe ausgewählt ist.

4. Verfahren nach Anspruch 3, wobei das nichtmenschliche Tier funktionsfähiges GRP54-Polypeptid exprimiert.

5. Verfahren nach Anspruch 3 oder 4, wobei das nichtmenschliche Tier ein nichtmenschliches Wildtyptier ist.

6. Verfahren nach Anspruch 3, 4 oder 5, wobei das nichtmenschliche Tier ein Nagetier, bevorzugt eine Maus, ist.

7. Verfahren zum Hervorrufen eines bei der Diagnose einer oder bei der Feststellung der Anfälligkeit für eine auf Sexualhormone bezogene Störung bei einem Einzelwesen nützlichen Symptoms, umfassend den Nachweis einer Veränderung im Expressionsmuster oder -spiegel eines GPR54-Polypeptids in einer Probe vom Einzelwesen.

8. Verfahren zum Hervorrufen eines bei der Diagnose einer oder bei der Feststellung der Anfälligkeit für eine auf Sexualhormone bezogen Störung bei einem Einzelwesen nützlichen Symptoms, umfassend den Nachweis eines Polymorphismus in einem GPR54-Polynukleotid in einer Probe vom Einzelwesen.

9. Verfahren nach einem der Ansprüche 1 und 3 bis 8, wobei der Agonist oder Antagonist ein Immunglobulin umfasst, bevorzugt eins, das spezifisch an ein GPR54-Polypeptid binden kann.

10. Verwendung eines transgenen nichtmenschlichen Tiers mit einem funktionell zerstörten endogenen GRP54-Gen oder einer isolierten Zelle oder von Gewebe von einem solchen in einem Verfahren zum Auffinden eines Agonisten oder Antagonisten eines GPR54-Polypeptids zur Behandlung von auf Sexualhormone bezogenen Störungen, zur Verwendung als Hormonersatztherapie (HRT), zur Beeinflussung der sexualhormonalen Achse eines Tieres oder zur Regulierung der Fruchtbarkeit und der Libido.

11. Verwendung nach Anspruch 10, wobei das transgene nichtmenschliche Tier ein Nagetier, bevorzugt eine Maus, ist.

12. Verwendung eines in einem der Ansprüche 10 und 11 definierten transgenen nichtmenschlichen Tiers oder einer isolierten Zelle oder von Gewebe von einem solchen als Modell für eine Fortpflanzungsstörung oder eine auf Sexualhormone bezogene Störung.

13. Verfahren oder Verwendung nach einem der vorangehenden Ansprüche, wobei die auf Sexualhormone bezogene Störung aus der aus Fruchtbarkeitsstörung und Störung der Libido, Regulierung der Fruchtbarkeit, Empfängnisverhütung, Libido und dem Einsetzens der Pubertät, Laktation, Osteoporose/Osteopetrose, Menopause einschließlich eines auf die Menopause bezogenen Hormonungleichgewichts, hormonabhängigem Krebs und gutartiger Prostatahypertrophie bestehenden Gruppe ausgewählt ist.

## Revendications

1. Procédé pour identifier une molécule, convenant au traitement d'un trouble associé aux hormones de reproduction, pour utilisation comme thérapie de substitution hormonale (HRT), pour manipuler l'axe des hormones sexuelles d'un animal ou pour moduler la fertilité et la libido, le procédé comprenant l'étape consistant à déterminer si une molécule candidate est un agoniste ou un antagoniste d'un polypeptide GPR54.

2. Utilisation d'un polypeptide GPR54 ou d'un polynucléotide pour l'identification d'une molécule convenant au traitement d'un trouble associé aux hormones de reproduction, pour utilisation comme thérapie de substitution hormonale (HRT), pour manipuler l'axe des hormones sexuelles d'un animal ou pour moduler la fertilité et la libido.

3. Procédé pour identifier un antagoniste d'un polypeptide GPR54, le procédé comprenant les étapes consistant à administrer une molécule candidate à un animal non humain et à déterminer si l'animal non humain présente un phénotype choisi dans le groupe constitué de : modifications de la morphologie des organes reproducteurs et des organes associés, incluant l'une quelconque des modifications suivantes : atrophie des glandes préputiales, micropénis, taille réduite des testicules, taille réduite des vésicules séminales, taille réduite des glandes coagulantes, masse musculaire réduite, masse de graisse brune réduite, taille réduite de l'estomac, glandes salivaires sous-maxillaires réduites, distance ano-génitale réduite ; modifications des taux et/ou des profils d'hormones de reproduction (incluant les cycles), incluant l'une quelconque des modifications suivantes : taux de testostérone réduits, taux d'oestrogènes réduits, taux de gonadotrophines réduits, taux réduits de l'hormone folliculostimulante (FSH) ; modifications du comportement sexuel/reproducteur, incluant le manque d'intérêt sexuel et le manque d'accouplement ; et la stérilité.

4. Procédé selon la revendication 3, dans lequel l'animal non humain exprime le polypeptide fonctionnel GPR54.

5. Procédé selon la revendication 3 ou 4, dans lequel l'animal non humain est un animal non humain de type sauvage.

6. Procédé selon la revendication 3, 4 ou 5, dans lequel l'animal non humain est un rongeur, de préférence une souris.

7. Procédé pour fournir une indication utile pour le diagnostic ou une détermination d'une prédisposition à un trouble associé aux hormones de reproduction chez un individu, le procédé comprenant la détection d'un changement dans le profil ou le taux d'expression d'un polypeptide GPR54 dans un échantillon prélevé sur l'individu.

8. Procédé pour fournir une indication utile pour le diagnostic ou une détermination d'une prédisposition à un trouble associé aux hormones de reproduction chez un individu, le procédé comprenant la détection d'un polymorphisme dans un polynucléotide codant pour le GRP54 dans un échantillon prélevé sur l'individu.

9. Procédé selon l'une quelconque des revendications 1 et 3 à 8, dans lequel l'agoniste ou l'antagoniste comprend une immunoglobuline, de préférence capable de se lier de manière spécifique à un polypeptide GPR54.

10. Utilisation d'un animal transgénique non humain ayant un gène codant pour le GPR54, endogène et interrompu au plan fonctionnel, ou d'une cellule ou d'un tissu isolés de celui-ci, dans un procédé d'identification d'un agoniste ou d'un antagoniste d'un polypeptide GPR54 pour le traitement d'un trouble associé aux hormones de reproduction, pour usage comme thérapie de substitution hormonale (HRT), pour manipuler l'axe des hormones sexuelles d'un animal ou pour moduler la fertilité et la libido.

11. Utilisation selon la revendication 10, selon laquelle l'animal transgénique non humain est un rongeur, de préférence une souris.

12. Utilisation d'un animal transgénique non humain selon l'une quelconque des revendications 10 ou 11, ou d'une cellule ou d'un tissu isolés de celui-ci, comme modèle d'un trouble de la reproduction ou d'un trouble associé aux hormones de reproduction.

13. Procédé ou utilisation selon l'une quelconque des revendications précédentes, dans lequel le trouble associé aux hormones de reproduction est choisi dans le groupe constitué de : un trouble de la fertilité et un trouble de la libido, une modulation de la fertilité, de la contraception, de la libido et de l'apparition de la puberté, de l'allaitement, de l'ostéoporose/ostéopétrose, de la ménopause, incluant un déséquilibre hormonal associé à la ménopause, un cancer hormono-dépendant et une hypertrophie bénigne de la prostate.
